# EUROPEAN PATENT APPLICATION

(11) **EP 4 549 571 A1**
(43) Date of publication of application: **07.05.2025**
(21) Application number: 23831109.6
(22) Date of filing: 14.06.2023
(51) Int. Cl.: C12N 15/60, C12N 1/15, C12N 1/19, C12N 1/21, C12N 5/10, C12N 9/88, C12N 15/63, C12P 13/02

(54) **MUTANT NITRILE HYDRATASE, NUCLEIC ACID ENCODING MUTANT NITRILE HYDRATASE, VECTOR AND TRANSFORMANT CONTAINING NUCLEIC ACID, PRODUCTION METHOD OF MUTANT NITRILE HYDRATASE, AND PRODUCTION METHOD OF AMIDE COMPOUND**

(30) Priority: 30.06.2022 JP 2022106069
(71) Applicant: Mitsui Chemicals, Inc., Tokyo 104-0028 (JP)
(72) Inventor: MOCHIZUKI, Daisuke, Mobara-shi, Chiba 297-0017 (JP); TOKUDA, Junko, Mobara-shi, Chiba 297-0017 (JP); KIDA, Yasushi, Mobara-shi, Chiba 297-0017 (JP)
(74) Representative: Mewburn Ellis LLP
(86) International application number: PCT/JP2023/022166
(87) International publication number: WO 2024/004661

(57) **Abstract**

A mutant nitrile hydratase, being a nitrile hydratase having an α subunit with a sequence identity of 90% or more with respect to an amino acid sequence represented by SEQ ID NO: 1 and a β subunit with a sequence identity of 90% or more with respect to an amino acid sequence represented by SEQ ID NO: 2, the nitrile hydratase including at least one substitution to a specific amino acid residue from an amino acid residue at a position selected from the group consisting of 23th, 105th, 142nd, 145th, 152th, 153rd, 180th and 188th from the N-terminus in the α subunit, and 89th, 122nd, 149th, 153rd, 189th, 207th, 232nd, 233rd and 50th from the N-terminus in the β subunit.

## Description

### Technical Field

The present disclosure relates to a mutant nitrile hydratase, a nucleic acid encoding the mutant nitrile hydratase, a vector and a transformant containing the nucleic acid, a production method of the mutant nitrile hydratase, and a production method of an amide compound.

### Background Art

Nitrile hydratase is an enzyme that exhibits a nitrile hydration activity; i.e., an activity that converts a nitrile group of various compounds into an amide group by hydration. Nitrile hydratase is used in the process of industrial production of amide compounds by way of enzymatic reaction.

It is known that acrylonitrile, which is used as a raw material in the production of acrylamide as an amide compound, may contain various impurities (such as acrolein as a byproduct). Patent Document 1 describes that acrolein is typically generated during the production of acrylonitrile, whereby an insoluble polymer is formed as a side reaction.

Patent Document 2 describes that acrolein, as an example of impurities included in acrylonitrile, inhibits the synthesis reaction of acrylamide by nitrile hydratase. Patent Document 2 describes that the inhibitive action of acrolein with respect to the catalytic action of nitrile hydratase may be avoided by controlling the concentration of acrolein in acrylonitrile as a raw material to 1 ppm or less using an ion exchange resin or the like.

### Prior Art Documents

### Patent Documents

Patent Document 1: Japanese Patent Application Laid-Open (JP-A) No. 2012-139233
Patent Document 2: Japanese Patent Application Laid-Open (JP-A) No. 2012-29695

### Summary of the Invention

### Problem to be Solved by the Invention

However, the method described in Patent Document 2 relates to avoiding the inhibitive action of acrolein with respect to nitrile hydratase activity by controlling the concentration of acrolein in acrylonitrile as a raw material to 1 ppm or less, rather than reducing the inhibition with respect to nitrile hydratase activity in the presence of impurities. Further, from the viewpoint of reducing production costs, a technique is desired by which the inhibition with respect to nitrile hydratase activity can be reduced without the need for a process to remove the impurities or an additive to trap the impurities in the reaction system.

In light of the foregoing, the present disclosure provides a technique regarding a novel mutant nitrile hydratase in which the inhibitive action of an impurity is reduced with respect to the generation of an amide compound using nitrile hydratase, even in the presence of the impurity.

The present disclosure includes the following embodiments.
<1> A mutant nitrile hydratase, being a nitrile hydratase having an α subunit with a sequence identity of 90% or more with respect to an amino acid sequence represented by SEQ ID NO: 1 and a β subunit with a sequence identity of 90% or more with respect to an amino acid sequence represented by SEQ ID NO: 2, the nitrile hydratase including at least one substitution of an amino acid residue selected from the group consisting of the following (a) to (q):
   (a) a substitution to Gly from Ala as an amino acid residue corresponding to a 23rd position from an N-terminus of SEQ ID NO: 1 in the α subunit;
   (b) a substitution to Met from Val as an amino acid residue corresponding to a 105th position from the N-terminus of SEQ ID NO: 1 in the α subunit;
   (c) a substitution to Cys from Leu as an amino acid residue corresponding to a 142th position from the N-terminus of SEQ ID NO: 1 in the α subunit;
   (d) a substitution to Asp from Glu as an amino acid residue corresponding to a 145th position from the N-terminus of SEQ ID NO: 1 in the α subunit;
   (e) a substitution to Gly from Pro as an amino acid residue at a 152nd position from the N-terminus of SEQ ID NO: 1 in the α subunit;
   (f) a substitution to Arg or Val from Pro as an amino acid residue corresponding to a 153rd position from the N-terminus of SEQ ID NO: 1 in the α subunit;
   (g) a substitution to Val from Gly as an amino acid residue corresponding to a 180th position from the N-terminus of SEQ ID NO: 1 in the α subunit;
   (h) a substitution to Leu from Glu as an amino acid residue corresponding to a 89th position from an N-terminus of SEQ ID NO: 2 in the β subunit;
   (i) a substitution to Ile, Leu or Thr from Ala as an amino acid residue corresponding to a 122nd position from the N-terminus of SEQ ID NO: 2 in the β subunit;
   (j) a substitution to Gln from Thr as an amino acid residue corresponding to a 149th position from the N-terminus of SEQ ID NO: 2 in the β subunit;
   (k) a substitution to Val, Arg or Pro from Lys as an amino acid residue corresponding to a 153rd position from the N-terminus of SEQ ID NO: 2 in the β subunit;
   (l) a substitution to Ser from Cys as an amino acid residue corresponding to a 189th position from the N-terminus of SEQ ID NO: 2 in the β subunit;
   (m) a substitution to Pro, Ser or Gly from Glu as an amino acid residue corresponding to a 207th position from the N-terminus of SEQ ID NO: 2 in the β subunit;
   (n) a substitution to Trp, Ser, Pro, Phe, Ile, Arg, Cys or Gly from Ala as an amino acid residue corresponding to a 232nd position from the N-terminus of SEQ ID NO: 2 in the β subunit;
   (o) a substitution to Glu or Gln from Ala as an amino acid residue corresponding to a 233rd position from the N-terminus of SEQ ID NO: 2 in the β subunit;
   (p) a substitution to Lys from Thr as an amino acid residue corresponding to a 188th position from the N-terminus of SEQ ID NO: 1 in the α subunit; and
   (q) a substitution to Gly from Glu as an amino acid residue corresponding to a 50th position from the N-terminus of SEQ ID NO: 2 in the β subunit.
<2> The mutant nitrile hydratase according to <1>, including at least two substitutions of an amino acid residue selected from the group consisting of (a) to (q).
<3> The mutant nitrile hydratase according to <1> or <2>, including at least one substitution of an amino acid residue selected from the group consisting of (i), (g), (j) and (n).
<4> The mutant nitrile hydratase according to <1> or <2>, including at least one substitution of an amino acid residue selected from the group consisting of (i), (g), (l) and (m).
<5> The mutant nitrile hydratase according to <1> or <2>, including at least one substitution of an amino acid residue selected from the group consisting of (g), (p) and (q).
<6> The mutant nitrile hydratase according to <1> or <2>, wherein the amino acid sequence of the α subunit further includes at least one substitution of an amino acid residue selected from the group consisting of the following (A1) to (A6):
   (A1) a substitution to Val from Arg as an amino acid residue corresponding to a 20th position from the N-terminus of SEQ ID NO: 1 in the α subunit;
   (A2) a substitution to Arg from Gln as an amino acid residue corresponding to a 141st position from the N-terminus of SEQ ID NO: 1 in the α subunit;
   (A3) a substitution to Ala from Glu as an amino acid residue corresponding to a 185th position from the N-terminus of SEQ ID NO: 1 in the α subunit;
   (A4) a substitution to Arg from Ala as an amino acid residue corresponding to a 187th position from the N-terminus of SEQ ID NO: 1 in the α subunit;
   (A5) a substitution to Ala from Pro as an amino acid residue corresponding to a 200th position from the N-terminus of SEQ ID NO: 1 in the α subunit; and
   (A6) a substitution to Leu from Val as an amino acid residue corresponding to a 204th position from the N-terminus of SEQ ID NO: 1 in the α subunit.
<7> The mutant nitrile hydratase according to <1> or <2>, wherein the amino acid sequence of the β subunit further includes at least one substitution of an amino acid residue selected from the group consisting of the following (B1) to (B9):
   (B1) a substitution to Ile from Thr as an amino acid residue corresponding to a 40th position from the N-terminus of SEQ ID NO: 2 in the β subunit;
   (B2) a substitution to Pro from Arg as an amino acid residue corresponding to a 42nd position from the N-terminus of SEQ ID NO: 2 in the β subunit;
   (B3) a substitution to Cys from Thr as an amino acid residue corresponding to a 76th position from the N-terminus of SEQ ID NO: 2 in the β subunit;
   (B4) a substitution to His from Thr as an amino acid residue corresponding to a 83rd position from the N-terminus of SEQ ID NO: 2 in the β subunit;
   (B5) a substitution to Arg, Pro or Ser from Leu as an amino acid residue corresponding to a 88th position from the N-terminus of SEQ ID NO: 2 in the β subunit;
   (B6) a substitution to Gln or Thr from Arg as an amino acid residue corresponding to a 146th position from the N-terminus of SEQ ID NO: 2 in the β subunit;
   (B7) a substitution to Gln or Ile from Arg as an amino acid residue corresponding to a 160th position from the N-terminus of SEQ ID NO: 2 in the β subunit;
   (B8) a substitution Arg from Glu as an amino acid residue corresponding to a 108th position from the N-terminus of SEQ ID NO: 2 in the β subunit; and
   (B9) a substitution to Cys from Tyr as an amino acid residue corresponding to a 176th position from the N-terminus of SEQ ID NO: 2 in the β subunit.
<8> A nucleic acid, encoding the mutant nitrile hydratase according to any one of <1> to <7>.
<9> A vector, including the nucleic acid according to <8>.
<10> The vector according to <9>, being an expression vector.
<11> A transformant, including the expression vector according to <10>.
<12> A method of producing a mutant nitrile hydratase, the method including:
   growing the transformant according to <11> in a culture medium; and
   collecting the mutant nitrile hydratase according to any one of <1> to <7> from at least one of the transformant or the culture medium.
<13> A mutant nitrile hydratase obtained by the production method according to claim <12>
<14> A method of producing an amide compound, the method including contacting the mutant nitrile hydratase according to any one of <1> to <7> with a nitrile compound.

### Effect of the Invention

According to the present disclosure, it is possible to provide a technique regarding a novel mutant nitrile hydratase, which achieves reduction in the inhibition by an impurity with respect to a reaction to generate an amide compound using nitrile hydratase, even in the presence of the impurity.

### Embodiments for Implementing the Invention

Embodiments for carrying out the present invention will now be described in detail. However, the invention is in no way limited to the following embodiments.

In a numerical range described in a stepwise manner, in the present disclosure, an upper limit value or a lower limit value described in one numerical range may be replaced with an upper limit value or a lower limit value in another numerical range described in a stepwise manner. Further, in a numerical range described in the present disclosure, the upper limit value or the lower limit value in the numerical range may be replaced with a value shown in the Examples.

In the present disclosure, a respective compound may include plural kinds of substances.

In the present disclosure, in a case in which a respective component in a composition includes plural kinds of substances, the content of the respective component refers to the total content of the plural kinds of substances present in the composition, unless otherwise specified.

In the present disclosure, the definition of the term "step" includes not only an independent step which is distinguishable from another step, but also a step which is not clearly distinguishable from another step, as long as the purpose of the step is achieved.

In the present disclosure, any numerical range described using the expression "... to ..." represents a range in which numerical values described before and after the "to" are included in the range as a minimum value and a maximum value, respectively.

In the present disclosure, in a case in which a respective component in a composition includes plural kinds of substances, the content of the respective component refers to the total content of the plural kinds of substances present in the composition, unless otherwise specified.

In the present disclosure, the "enzymatic activity" refers to nitrile hydration activity by which a nitrile group is converted into an amide group.

In the present disclosure, when a nucleic acid chain forms a double-chain structure, explanation of a nucleotide sequence retained by the nucleic acid chain may be given based on a sequence of either one of the chains forming the double-chain structure. In that case, explanation of a sequence of the other chain should be applied as the complementary sequence.

In the present disclosure, the three alphabet letters indicate an amino acid residue, unless otherwise specified. For example, "Ile" indicates a residue of isoleucine.

### <Mutant nitrile hydratase>

The present disclosure provides a mutant nitrile hydratase, being a nitrile hydratase having an α subunit with a sequence identity of 90% or more with respect to an amino acid sequence represented by SEQ ID NO: 1 and a β subunit with a sequence identity of 90% or more with respect to an amino acid sequence represented by SEQ ID NO: 2, the nitrile hydratase including at least one substitution of an amino acid residue selected from the group consisting of the following (a) to (q):
(a) a substitution to Gly from Ala as an amino acid residue corresponding to a 23rd position from an N-terminus of SEQ ID NO: 1 in the α subunit;
(b) a substitution to Met from Val as an amino acid residue corresponding to a 105th position from the N-terminus of SEQ ID NO: 1 in the α subunit;
(c) a substitution to Cys from Leu as an amino acid residue corresponding to a 142th position from the N-terminus of SEQ ID NO: 1 in the α subunit;
(d) a substitution to Asp from Glu as an amino acid residue corresponding to a 145th position from the N-terminus of SEQ ID NO: 1 in the α subunit;
(e) a substitution to Gly from Pro as an amino acid residue at a 152nd position from the N-terminus of SEQ ID NO: 1 in the α subunit;
(f) a substitution to Arg or Val from Pro as an amino acid residue corresponding to a 153rd position from the N-terminus of SEQ ID NO: 1 in the α subunit;
(g) a substitution to Val from Gly as an amino acid residue corresponding to a 180th position from the N-terminus of SEQ ID NO: 1 in the α subunit;
(h) a substitution to Leu from Glu as an amino acid residue corresponding to a 89th position from an N-terminus of SEQ ID NO: 2 in the β subunit;
(i) a substitution to Ile, Leu or Thr from Ala as an amino acid residue corresponding to a 122nd position from the N-terminus of SEQ ID NO: 2 in the β subunit;
(j) a substitution to Gln from Thr as an amino acid residue corresponding to a 149th position from the N-terminus of SEQ ID NO: 2 in the β subunit;
(k) a substitution to Val, Arg or Pro from Lys as an amino acid residue corresponding to a 153rd position from the N-terminus of SEQ ID NO: 2 in the β subunit;
(l) a substitution to Ser from Cys as an amino acid residue corresponding to a 189th position from the N-terminus of SEQ ID NO: 2 in the β subunit;
(m) a substitution to Pro, Ser or Gly from Glu as an amino acid residue corresponding to a 207th position from the N-terminus of SEQ ID NO: 2 in the β subunit;
(n) a substitution to Trp, Ser, Pro, Phe, Ile, Arg, Cys or Gly from Ala as an amino acid residue corresponding to a 232nd position from the N-terminus of SEQ ID NO: 2 in the β subunit;
(o) a substitution to Glu or Gln from Ala as an amino acid residue corresponding to a 233rd position from the N-terminus of SEQ ID NO: 2 in the β subunit;
(p) a substitution to Lys from Thr as an amino acid residue corresponding to a 188th position from the N-terminus of SEQ ID NO: 1 in the α subunit; and
(q) a substitution to Gly from Glu as an amino acid residue corresponding to a 50th position from the N-terminus of SEQ ID NO: 2 in the β subunit.

The mutant nitrile hydratase including at least one substitution of an amino acid residue selected from the group consisting of (a) to (q) is a novel mutant that has not been previously reported.

Unlike nitrile hydratase derived from wild-type Pseudonocardia thermophila or conventional mutants thereof, the mutant nitrile hydratase according to the present disclosure exhibits an excellent enzymatic activity in the synthesis reaction of an amide compound from a nitrile compound, by reducing the inhibitive action of an impurity (such as acrolein) with respect to the generation of an amide compound using nitrile hydratase, even in the presence of the impurity.

Further, the effect of reducing the inhibition by an impurity, achieved by the mutant nitrile hydratase according to the present disclosure, is especially significant when the concentration of the impurity is high (for example, 1000 ppm or more). Therefore, according to the mutant nitrile hydratase of the present disclosure, it is possible to promote the reaction of synthesizing an amide compound in the industrial production using nitrile hydratase, even when the raw material is not sufficiently purified. As such, according to the mutant nitrile hydratase of the present disclosure, it is possible to achieve a remarkable improvement in the efficiency of industrial production of the amide compound, as compared with a case in which conventional enzymes are used.

The mutant nitrile hydratase of the present disclosure is a mutant nitrile hydratase that has an α subunit with a sequence identity of 90% or more with respect to an amino acid sequence represented by SEQ ID NO: 1 and a β subunit with a sequence identity of 90% or more with respect to an amino acid sequence represented by SEQ ID NO: 2, and includes at least one substitution of an amino acid residue selected from the group consisting of the foregoing (a) to (q).

The amino acid sequence represented by SEQ ID NO: 1 refers to an amino acid sequence of an α subunit of nitrile hydratase derived from wild-type Pseudonocardia thermophila.

The amino acid sequence represented by SEQ ID NO: 2 refers to an amino acid sequence of a β subunit of nitrile hydratase derived from wild-type Pseudonocardia thermophila.

The α subunit with a sequence identity of 90% or more with respect to an amino acid sequence represented by SEQ ID NO: 1 refers to an α subunit having an amino acid sequence with a sequence identity of 90% or more with respect to an amino acid sequence of an α subunit of nitrile hydratase derived from wild-type Pseudonocardia thermophila (hereinafter, also referred to as wild-type α subunit).

The β subunit with a sequence identity of 90% or more with respect to an amino acid sequence represented by SEQ ID NO: 2 refers to a β subunit having an amino acid sequence with a sequence identity of 90% or more with respect to an amino acid sequence of a β subunit of nitrile hydratase derived from wild-type Pseudonocardia thermophila (hereinafter, also referred to as wild-type β subunit).

The mutant nitrile hydratase having an α subunit with a sequence identity of 90% or more with respect to an amino acid sequence represented by SEQ ID NO: 1 may have an α subunit with a sequence identity of 95% or more with respect to an amino acid sequence represented by SEQ ID NO: 1, or may have an α subunit with a sequence identity of 98% or more with respect to an amino acid sequence represented by SEQ ID NO: 1.

The mutant nitrile hydratase having a βsubunit with a sequence identity of 90% or more with respect to an amino acid sequence represented by SEQ ID NO: 2 may have an β subunit with a sequence identity of 95% or more with respect to an amino acid sequence represented by SEQ ID NO: 2, or may have a β subunit with a sequence identity of 98% or more with respect to an amino acid sequence represented by SEQ ID NO: 2.

The mutant nitrile hydratase is a tetramer that is formed from dimers associating with each other, and each of the dimers forms a basic structural unit in which an α subunit and a β subunit are associated with each other.

In the mutant nitrile hydratase, the cysteine residue at the 111st position and the cysteine residue at the 113rd position from the N-terminus in the α subunit are converted into cysteine sulfinic acid (Cys-SOOH) and cysteine sulfenic acid (Cys-SOH) by posttranslation modification, respectively. Via these modified amino acid residues, the polypeptide chain of the α subunit is bound to a cobalt atom, thereby forming the active center.

### (Mutation site)

The mutant nitrile hydratase of the present disclosure includes at least one substitution of an amino acid residue selected from the group consisting of (a) to (q).

The mutant nitrile hydratase of the present disclosure may include multiple substitutions in combination, as long as it includes at least one substitution of an amino acid residue selected from the group consisting of (a) to (q).

The mutant nitrile hydratase of the present disclosure may include a substitution of an amino acid residue other than those selected from the group consisting of (a) to (q), as long as the inhibition by an impurity is reduced with respect to the reaction to generate an amide compound using nitrile hydratase.

From the viewpoint of further reducing the inhibition by an impurity with respect to a reaction to generate an amide compound using nitrile hydratase, the mutant nitrile hydratase of the present disclosure preferably includes at least two substitutions of an amino acid residue selected from the group consisting of (a) to (q).

From the viewpoint of further reducing the inhibition by an impurity with respect to the reaction to generate an amide compound using nitrile hydratase, the mutant nitrile hydratase of the present disclosure preferably includes an amino acid residue substitution of (i).

From the viewpoint of further reducing the inhibition by an impurity with respect to the reaction to generate an amide compound using nitrile hydratase, the mutant nitrile hydratase of the present disclosure may include at least 3, at least 4, at least 5, at least 6, at least 7, at least 8, at least 9, at least 10, at least 11, at least 12, at least 13, at least 14, at least 15, at least 16, or 17 of the amino acid residue substitution selected from the group consisting of (a) to (q).

The mutant nitrile hydratase of the present disclosure may include 16 or less, 15 or less, 14 or less, 13 or less, 12 or less, 11 or less, 10 or less, 9 or less, 8 or less, 7 or less, 6 or less, 5 or less, 4 or less, 3 or less, or 2 or less of the amino acid residue substitution selected from the group consisting of (a) to (q).

When the mutant nitrile hydratase of the present disclosure includes two of the amino acid residue substitution selected from the group consisting of (a) to (q), the combination thereof is not particularly limited.

For example, the mutant nitrile hydratase may include a combination of amino acid residue substitutions as described below, from the viewpoint of further reducing the inhibition by an impurity with respect to the reaction to generate an amide compound by nitrile hydratase.

In the following exemplary combinations, only the mutation sites corresponding to the amino acid residue substitution selected from the group consisting of (a) to (q) are indicated for simplification. For example, the combination of (i) β149 and (n) β232 refers to the combination of (i) a substitution from to Gly from Thr as an amino acid residue corresponding to the 149th position from the N-terminus in the β subunit in SEQ ID NO: 2 and (n) a substitution to Trp, Ser, Pro, Phe, Ile, Arg, Cys or Gly from Ala as an amino acid residue corresponding to the 232nd position from the N-terminus in the β subunit in SEQ ID NO: 2.

When there are multiple alternatives for the amino acid residue after the mutation, as in the case of (n), the combination may include any of the alternatives. The same applies to the exemplary combinations including three or more amino acid residue substitutions, as described later.
A combination of (j) β149 and (n) β232;
A combination of (i) β122 and (g) α180;
A combination of (l) β189 and (m) β207;
A combination of (o) β233 and (h) β89;
A combination of (b) α105 and (c) α142;
A combination of (c) α142 and (g) α180;
A combination of (i) β122 and (n) β232;
A combination of (i) β122 and (j) β149;
A combination of (q) β50 and (h) β89;
A combination of (p) α188 and (i) β189; and
A combination of (p) α188 and (q) β50.

When the mutant nitrile hydratase of the present disclosure includes three amino acid residue substitutions selected from the group consisting of (a) to (q) in combination, the combination thereof is not particularly limited. For example, from the viewpoint of further reducing the inhibition by an impurity with respect to the reaction to generate an amide compound using nitrile hydratase, the mutant nitrile hydratase may include the following combinations. In the following exemplary combinations, only the substitution sites are simply mentioned for the amino acid residue substitutions selected from the group consisting of (a) to (q).
A combination of (i) β122, (n) β232 and (g) α180;
A combination of (i) β122, (j) β149 and (n) β232;
A combination of (m) β207, (b) α105 and (g) α180;
A combination of (m) β207, (l) β189 and (g) α180;
A combination of (m) β207, (n) β232 and (g) α180;
A combination of (g) α180, (p) α188 and (q) β50;
A combination of (i) β122, (j) β149 and (m) β207;
A combination of (i) β122, (l) β189 and (m) β207; and
A combination of (q) β50, (a) α23 and (b) α105.

When the mutant nitrile hydratase of the present disclosure includes four amino acid residue substitutions selected from the group consisting of (a) to (q) in combination, the combination thereof is not particularly limited. For example, from the viewpoint of further reducing the inhibition by an impurity with respect to the reaction to generate an amide compound using nitrile hydratase, the mutant nitrile hydratase may include the following combinations. In the following exemplary combinations, only the substitution sites are simply mentioned for the amino acid residue substitutions selected from the group consisting of (a) to (q).
A combination of (i) β122, (j) β149, (n) β232 and (g) α180;
A combination of (i) β122, (m) β207, (n) β232 and (g) α180;
A combination of (i) β122, (l) β189, (m) β207 and (g) α180;
A combination of (p) α188, (q) β50, (a) α23 and (l) β189;
A combination of (p) α188, (q) β50, (g) α180 and (o) β233;
A combination of (a) α23, (d) α145, (e) α152 and (o) β233;
A combination of (b) α105, (f) α153, (h) β89 and (n) β232; and
A combination of (q) β50, (d) α145, (e) α152 and (o) β233.

As a matter of course, the mutant nitrile hydratase of the present disclosure may include five or more amino acid residue substitutions selected from the group consisting of (a) to (q) in combination.

When the mutant nitrile hydratase of the present disclosure includes at least one amino acid residue substitution selected from the group consisting of (i), (g), (l) and (m), thermal stability of nitrile hydratase tends to improve, in addition to an effect of suppressing the inhibition by an impurity. Conventional nitrile hydratases tend to reduce the catalytic reaction thereof at a temperature of higher than room temperature (20°C). Therefore, when a raw material that is less soluble at room temperature is used for the reaction, there have been problems that need to be solved in terms of production costs, such as insufficient yield of amide compound or the use of a cooling device during the production.

In this regard, the mutant nitrile hydratase of the present disclosure satisfying the foregoing conditions is highly stable and highly likely to maintain the catalytic activity even at a higher temperature (for example, 30°C) than room temperature (for example, 20°C) as compared with conventional nitrile hydratases. Accordingly, the mutant nitrile hydratase of the present disclosure can increase the temperature in the reaction system even in a case of using a raw material with a low solubility at room temperature. Further, the mutant nitrile hydratase of the present disclosure achieves a side benefit in terms of production costs as compared with conventional nitrile hydratases, by increasing the yield of the amide compound or by eliminating the need for a cooling device.

The mutant nitrile hydratase of the present disclosure may include at least one amino acid residue substitution selected from the group consisting of the following (A1) to (A6), from the viewpoint of further reducing the inhibition by an impurity with respect to the reaction to generate an amide compound using nitrile hydratase:
(A1) a substitution to Val from Arg as an amino acid residue corresponding to a 20th position from the N-terminus of SEQ ID NO: 1 in the α subunit;
(A2) a substitution to Arg from Gln as an amino acid residue corresponding to a 141st position from the N-terminus of SEQ ID NO: 1 in the α subunit;
(A3) a substitution to Ala from Glu as an amino acid residue corresponding to a 185th position from the N-terminus of SEQ ID NO: 1 in the α subunit;
(A4) a substitution to Arg from Ala as an amino acid residue corresponding to a 187th position from the N-terminus of SEQ ID NO: 1 in the α subunit;
(A5) a substitution to Ala from Pro as an amino acid residue corresponding to a 200th position from the N-terminus of SEQ ID NO: 1 in the α subunit; and
(A6) a substitution to Leu from Val as an amino acid residue corresponding to a 204th position from the N-terminus of SEQ ID NO: 1 in the α subunit.

The mutant nitrile hydratase of the present disclosure may include at least one amino acid residue substitution selected from the group consisting of the following (B1) to (B9), from the viewpoint of further reducing the inhibition by an impurity with respect to the reaction to generate an amide compound using nitrile hydratase:
(B1) a substitution to Ile from Thr as an amino acid residue corresponding to a 40th position from the N-terminus of SEQ ID NO: 2 in the β subunit;
(B2) a substitution to Pro from Arg as an amino acid residue corresponding to a 42nd position from the N-terminus of SEQ ID NO: 2 in the β subunit;
(B3) a substitution to Cys from Thr as an amino acid residue corresponding to a 76th position from the N-terminus of SEQ ID NO: 2 in the β subunit;
(B4) a substitution to His from Thr as an amino acid residue corresponding to a 83rd position from the N-terminus of SEQ ID NO: 2 in the β subunit;
(B5) a substitution to Arg, Pro or Ser from Leu as an amino acid residue corresponding to a 88th position from the N-terminus of SEQ ID NO: 2 in the β subunit;
(B6) a substitution to Gln or Thr from Arg as an amino acid residue corresponding to a 146th position from the N-terminus of SEQ ID NO: 2 in the β subunit;
(B7) a substitution to Gln or Ile from Arg as an amino acid residue corresponding to a 160th position from the N-terminus of SEQ ID NO: 2 in the β subunit;
(B8) a substitution Arg from Glu as an amino acid residue corresponding to a 108th position from the N-terminus of SEQ ID NO: 2 in the β subunit; and
(B9) a substitution to Cys from Tyr as an amino acid residue corresponding to a 176th position from the N-terminus of SEQ ID NO: 2 in the β subunit.

The mutant nitrile hydratase of the present disclosure may include an amino acid residue substitution other than the amino acid residue substitutions selected from the foregoing (a) to (q); the amino acid residue substitutions selected from the foregoing (A1) to (A6); and the amino acid residue substitutions selected from the foregoing (B1) to (B9), hereinafter referred to as the "other amino acid residue substitution", as long as the effect of the present disclosure is achieved.

In the mutant nitrile hydratase of the present disclosure, the total number of the amino acid residue substitution selected from the group consisting of (a) to (q), the group consisting of (A1) to (A6) and the group consisting of (B1) to (B9) is not particularly limited, as long as it is 1 or more. For example, the foregoing total number may be any one of 1 to 20, 1 to 15, 1 to 10, 1 to 8, 1 to 7, 1 to 5, 1 to 4, 1 to 3, 1 to 2, or 1.

From the viewpoint of further reducing the inhibition by an impurity with respect to the reaction to generate an amide compound using nitrile hydratase, the mutant nitrile hydratase may include at least one amino acid residue substitution selected from the foregoing (i), (g), (j) and (n); at least one amino acid residue substitution selected from the foregoing (i), (j) and (n); or each of the amino acid residue substitutions (i), (g), (j) and (n).

As an embodiment, from the viewpoint of further reducing the inhibition by an impurity with respect to the reaction to generate an amide compound using nitrile hydratase, and from the viewpoint of achieving excellent thermal stability, for example, the mutant nitrile hydratase may include at least one amino acid residue substitution selected from the foregoing (i), (g), (l) and (m); at least one amino acid residue substitution selected from the foregoing (i), (l) and (m); or each of the amino acid residue substitutions (i), (g), (l) and (m).

As an embodiment, from the viewpoint of further reducing the inhibition by an impurity with respect to the reaction to generate an amide compound using nitrile hydratase, for example, the mutant nitrile hydratase may include at least one amino acid residue substitution selected from the foregoing (g), (p) and (q); at least one amino acid residue substitution selected from the foregoing (p) and (q); or each of the amino acid residue substitutions (g), (p) and (q).

The following are exemplary combinations of two amino acid residue substitutions selected from the group consisting of (a) to (q), the group consisting of (A1) to (A6), and the group consisting of (B1) to (B9), while the present disclosure is not limited thereto. In the table, the numbers in the column indicating the substitution sites refer to a position from the N-terminus in an amino acid sequence of the corresponding subunit.

**Table 1-1**

| No. | Mutation site | Before mutation | After mutation |
|---|---|---|---|
| 2-1 | β122 | Ala | Ile |
| | β232 | Ala | Trp |
| 2-2 | β122 | Ala | Ile |
| | β153 | Lys | Arg |
| 2-3 | β207 | Glu | Pro |
| | α180 | Gly | Val |
| 2-4 | α180 | Gly | Val |
| | β122 | Ala | Ile |
| 2-5 | β122 | Ala | Ile |
| | β149 | Thr | Gln |
| 2-6 | β149 | Thr | Gln |
| | β232 | Ala | Trp |
| 2-7 | β189 | Cys | Ser |
| | β207 | Glu | Pro |
| 2-8 | β122 | Ala | Ile |
| | β189 | Cys | Ser |
| 2-9 | β122 | Ala | Ile |
| | β207 | Glu | Pro |
| 2-10 | α180 | Gly | Val |
| | β149 | Thr | Gln |
| 2-11 | α188 | Thr | Lys |
| | β50 | Glu | Gly |
| 2-12 | α23 | Ala | Gly |
| | α142 | Leu | Cys |
| 2-13 | α145 | Glu | Asp |
| | α152 | Pro | Gly |
| 2-14 | β89 | Glu | Leu |
| | β122 | Ala | Leu |
| 2-15 | β153 | Lys | Val |
| | β233 | Ala | Glu |

**Table 1-2**

| No. | Mutation site | Before mutation | After mutation |
|---|---|---|---|
| 2-16 | α23 | Ala | Gly |
| | α105 | Val | Met |
| 2-17 | α188 | Thr | Lys |
| | α185 | Glu | Ala |
| 2-18 | β50 | Glu | Gly |
| | β76 | Thr | Cys |
| 2-19 | α188 | Thr | Lys |
| | α20 | Arg | Val |
| 2-20 | β233 | Ala | Glu |
| | α23 | Ala | Gly |
| 2-21 | β153 | Lys | Val |
| | α105 | Val | Met |
| 2-22 | α152 | Pro | Gly |
| | α141 | Gln | Arg |
| 2-23 | α153 | Pro | Arg |
| | α145 | Glu | Asp |
| 2-24 | β122 | Ala | Ile |
| | α188 | Thr | Lys |
| 2-25 | β232 | Ala | Trp |
| | α200 | Pro | Ala |
| 2-26 | α23 | Ala | Gly |
| | α204 | Val | Leu |
| 2-27 | α142 | Leu | Cys |
| | β40 | Thr | Ile |
| 2-28 | α180 | Gly | Val |
| | β42 | Arg | Pro |
| 2-29 | α180 | Gly | Val |
| | β50 | Glu | Gly |
| 2-30 | β232 | Ala | Trp |
| | β160 | Arg | Gln |

The following are exemplary combinations of three amino acid residue substitutions selected from the group consisting of (a) to (q), the group consisting of (A1) to (A6), and the group consisting of (B1) to (B9), while the present disclosure is not limited thereto. In the table, the numbers in the column indicating the substitution sites refer to a position from the N-terminus in an amino acid sequence of the corresponding subunit.

**Table 2-1**

| No. | Mutation site | Before mutation | After mutation |
|---|---|---|---|
| 3-1 | β122 | Ala | Leu |
| | β232 | Ala | Pro |
| | α180 | Gly | Val |
| 3-2 | β122 | Ala | Ile |
| | β149 | Thr | Gln |
| | β232 | Ala | Trp |
| 3-3 | β207 | Glu | Gly |
| | α105 | Val | Met |
| | α180 | Gly | Val |
| 3-4 | β207 | Glu | Gly |
| | β189 | Cys | Ser |
| | α180 | Gly | Val |
| 3-5 | β207 | Glu | Gly |
| | β232 | Ala | Gly |
| | α180 | Gly | Val |
| 3-6 | β207 | Glu | Gly |
| | β232 | Ala | Arg |
| | α180 | Gly | Val |
| 3-7 | β122 | Ala | Ile |
| | β149 | Thr | Gln |
| | β207 | Glu | Gly |
| 3-8 | β122 | Ala | Ile |
| | β207 | Glu | Gly |
| | β232 | Ala | Arg |
| 3-9 | β122 | Ala | Ile |
| | β189 | Cys | Ser |
| | β207 | Glu | Gly |
| 3-10 | α23 | Ala | Gly |
| | α180 | Gly | Val |
| | β122 | Ala | Ile |

**Table 2-2**

| No. | Mutation site | Before mutation | After mutation |
|---|---|---|---|
| 3-11 | α105 | Val | Met |
| | α142 | Leu | Cys |
| | α180 | Gly | Val |
| 3-12 | β149 | Thr | Gln |
| | β153 | Lys | Val |
| | β233 | Ala | Glu |
| 3-13 | α180 | Gly | Val |
| | β189 | Cys | Ser |
| | β233 | Ala | Glu |
| 3-14 | α188 | Thr | Lys |
| | β50 | Glu | Gly |
| | α180 | Gly | Val |
| 3-15 | β50 | Glu | Gly |
| | α23 | Ala | Gly |
| | α204 | Val | Leu |
| 3-16 | β149 | Thr | Gln |
| | β207 | Glu | Gly |
| | α204 | Val | Leu |
| 3-17 | β233 | Ala | Glu |
| | α105 | Val | Met |
| | α185 | Glu | Ala |
| 3-18 | β189 | Cys | Ser |
| | β149 | Thr | Gln |
| | β232 | Ala | Trp |
| 3-19 | α23 | Ala | Gly |
| | α185 | Glu | Ala |
| | α204 | Val | Leu |
| 3-20 | β89 | Glu | Leu |
| | β146 | Arg | Gln |
| | β160 | Arg | Gln |

The following are exemplary combinations of four amino acid residue substitutions selected from the group consisting of (a) to (q), the group consisting of (A1) to (A6), and the group consisting of (B1) to (B9), while the present disclosure is not limited thereto. In the table, the numbers in the column indicating the substitution sites refer to a position from the N-terminus in an amino acid sequence of the corresponding subunit.

**Table 3**

| No. | Mutation site | Before mutation | After mutation |
|---|---|---|---|
| 4-1 | β122 | Ala | Leu |
| | β149 | Thr | Gln |
| | β232 | Ala | Cys |
| | α180 | Gly | Val |
| 4-2 | β122 | Ala | Ile |
| | β207 | Glu | Gly |
| | β232 | Ala | Trp |
| | α180 | Gly | Val |
| 4-3 | β122 | Ala | Ile |
| | β189 | Cys | Ser |
| | β207 | Glu | Gly |
| | α180 | Gly | Val |
| 4-4 | α188 | Thr | Lys |
| | β50 | Glu | Gly |
| | α23 | Ala | Gly |
| | β189 | Cys | Ser |
| 4-5 | α188 | Thr | Lys |
| | β50 | Glu | Gly |
| | α180 | Gly | Val |
| | β233 | Ala | Glu |
| 4-6 | α105 | Val | Met |
| | β153 | Lys | Arg |
| | α180 | Gly | Val |
| | β89 | Glu | Leu |
| 4-7 | α23 | Ala | Gly |
| | α145 | Glu | Asp |
| | β149 | Thr | Gln |
| | β233 | Ala | Glu |
| 4-8 | α152 | Pro | Gly |
| | α153 | Pro | Arg |
| | β89 | Glu | Leu |
| | β153 | Lys | Val |
| 4-9 | β233 | Ala | Glu |
| | α105 | Val | Met |
| | β50 | Glu | Gly |
| | β160 | Arg | Gln |
| 4-10 | β153 | Lys | Arg |
| | α141 | Gln | Arg |
| | α200 | Pro | Ala |
| | β42 | Arg | Pro |
| 4-11 | β89 | Glu | Leu |
| | β76 | Thr | Cys |
| | β88 | Leu | Arg |
| | β146 | Arg | Gln |

The following are exemplary combinations of five amino acid residue substitutions selected from the group consisting of (a) to (q), the group consisting of (A1) to (A6), and the group consisting of (B1) to (B9), while the present disclosure is not limited thereto. In the table, the numbers in the column indicating the substitution sites refer to a position from the N-terminus in an amino acid sequence of the corresponding subunit.

**Table 4**

| No. | Mutation site | Before mutation | After mutation |
|---|---|---|---|
| 5-1 | β122 | Ala | Ile |
| | β189 | Cys | Ser |
| | β207 | Glu | Gly |
| | β232 | Ala | Trp |
| | α180 | Gly | Val |
| 5-2 | β122 | Ala | Ile |
| | β176 | Tyr | Cys |
| | β189 | Cys | Ser |
| | β207 | Glu | Gly |
| | α180 | Gly | Val |
| 5-3 | α188 | Thr | Lys |
| | β50 | Glu | Gly |
| | α180 | Gly | Val |
| | β146 | Arg | Gln |
| | β160 | Arg | Gln |
| 5-4 | α23 | Ala | Gly |
| | α180 | Gly | Val |
| | β83 | Thr | His |
| | β108 | Glu | Arg |
| | β176 | Tyr | Cys |

### Nitrile hydratase

The origin of the mutant nitrile hydratase of the present disclosure is not particularly limited, as long as it has an α subunit with a sequence identity of 90% or more with respect to an amino acid sequence represented by SEQ ID NO: 1 and a β subunit with a sequence identity of 90% or more with respect to an amino acid sequence represented by SEQ ID NO: 2, and includes at least one substitution of an amino acid residue selected from the group consisting of the foregoing (a) to (q).

For example, the origin of the nitrile hydratase may be nitrile hydratase derived from wild-type Pseudonocardia thermophila, or may be a mutant of nitrile hydratase derived from wild-type Pseudonocardia thermophila.

### (Mutant nitrile hydratase)

The mutant nitrile hydratase includes a mutant nitrile hydratase obtained by subjecting nitrile hydratase derived from wild-type Pseudonocardia thermophila to mutation selected from the group consisting of the following (i) to (v):
(i) substitution of one or more amino acid residues to a different amino acid residue;
(ii) defection of one or more amino acid residues, except the amino acid residue substitutions of (a) to (q);
(iii) insertion of an amino acid residue;
(iv) addition of an amino acid residue to either one or both of the N-terminus and the C-terminus in the α subunit; and
(v) addition of an amino acid residue to either one or both of the N-terminus and the C-terminus in the β subunit.

In the α subunit of the mutant nitrile hydratase, the number of substitution of amino acid residue is, for example, from 1 to 20, from 1 to 15, from 1 to 10, from 1 to 8, from 1 to 7, from 1 to 6, from 1 to 5, from 1 to 4, from 1 to 3, from 1 to 2, or 1. The number of substitution of amino acid residue may be zero.

In the β subunit of the mutant nitrile hydratase, the number of substitution of amino acid residue is, for example, from 1 to 20, from 1 to 15, from 1 to 10, from 1 to 8, from 1 to 7, from 1 to 6, from 1 to 5, from 1 to 4, from 1 to 3, from 1 to 2, or 1. The number of substitution of amino acid residue may be zero.

In the α subunit of the mutant nitrile hydratase, the number of defection of amino acid residue is, for example, from 1 to 10, from 1 to 7, from 1 to 4, from 1 to 2, or 1. The number of defection of amino acid residue may be zero.

In the β subunit of the mutant nitrile hydratase, the number of defection of amino acid residue is, for example, from 1 to 10, from 1 to 7, from 1 to 4, from 1 to 2, or 1. The number of defection of amino acid residue may be zero.

In the α subunit of the mutant nitrile hydratase, the number of insertion of amino acid residue is, for example, from 1 to 10, from 1 to 7, from 1 to 4, from 1 to 2, or 1. The number of insertion of amino acid residue may be zero.

In the β subunit of the mutant nitrile hydratase, the number of insertion of amino acid residue is, for example, from 1 to 10, from 1 to 7, from 1 to 4, from 1 to 2, or 1. The number of insertion of amino acid residue may be zero.

In the α subunit of the mutant nitrile hydratase, the total number of substitution, defection or insertion of amino acid residue is, for example, from 1 to 20, from 1 to 14, from 1 to 8, from 1 to 4, from 1 to 2, or 1. In cases in which an amino acid residue is added at the terminus, or the like, the total number of substitution, defection or insertion of amino acid residue may be zero.

In the β subunit of the mutant nitrile hydratase, the total number of substitution, defection or insertion of amino acid residue is, for example, from 1 to 20, from 1 to 14, from 1 to 8, from 1 to 4, from 1 to 2, or 1. In cases in which an amino acid residue is added at the terminus, or the like, the total number of substitution, defection or insertion of amino acid residue may be zero.

In the α subunit of the mutant nitrile hydratase, the number of addition of amino acid residue per terminus is, for example, from 1 to 60, from 1 to 40, from 1 to 20, from 1 to 10, from 1 to 5, from 1 to 3, or 1. The number of addition of amino acid residue per terminus may be zero.

In the β subunit of the mutant nitrile hydratase, the number of addition of amino acid residue per terminus is, for example, from 1 to 60, from 1 to 40, from 1 to 20, from 1 to 10, from 1 to 5, from 1 to 3, or 1. The number of addition of amino acid residue per terminus may be zero.

The amino acid residue to be added at a terminus may be a secretory signal sequence, for example. The amino acid residue may be added at either the N-terminus or the C-terminus alone, or may be added at each of the N-terminus and the C-terminus.

The analogy between the mutant nitrile hydratase and nitrile hydratase derived from wild-type Pseudonocardia thermophila may be expressed by the sequence identity.

For example, the amino acid sequence of the α subunit of the mutant nitrile hydratase has a sequence identity of 90% or more, 95% or more, 96% or more, 97% or more, 98% or more, or 99 % or more, with respect to an amino acid sequence represented by SEQ ID NO: 1.

For example, the amino acid sequence of the β subunit of the mutant nitrile hydratase has a sequence identity of 90% or more, 95% or more, 96% or more, 97% or more, 98% or more, or 99 % or more, with respect to an amino acid sequence represented by SEQ ID NO: 2.

The alignment of the sequences may be performed using Clustal W (1.83) with initial parameters (including gap open penalty: 10, gap extension penalty: 0.05). The sequence identity is calculated based on the full length of nitrile hydratase.

When the amino acid sequence of the mutant nitrile hydratase and the amino acid sequence of nitrile hydratase derived from wild-type Pseudonocardia thermophila are subjected to alignment, there are cases in which the amino acid residues corresponding to each other may be located at different distances from the N-terminus of the subunit as specified above.

In such cases, in the present disclosure, the amino acid residue at the Xth position from the N-terminus in the subunit of the mutant nitrile hydratase is regarded as an amino acid residue that corresponds to an amino acid residue at the Xth position from the N-terminus in the subunit of nitrile hydratase derived from wild-type Pseudonocardia thermophila, in terms of alignment.

For example, the "amino acid residue at the 23rd position from the N-terminus of the α subunit", the "Ala residue at the 23rd position from the N-terminus of the α subunit" or the "Ala residue as an amino acid residue at the 23rd position from the N-terminus of the α subunit" may be located at a position other than the 23rd position from the N-terminus of the α subunit.

For example, there may be cases in which the amino acid residue at the 23rd position from the N-terminus of the α subunit (not necessarily Ala) corresponds to Ala residue as an amino acid residue at the 24th position from the N-terminus of the α subunit of nitrile hydratase derived from wild-type Pseudonocardia thermophila, as a result of insertion of an amino acid residue. In such cases, the "amino acid residue at the 23rd position from the N-terminus of the α subunit", the "Ala residue at the 23rd position from the N-terminus of the α subunit" or the "Ala residue as an amino acid residue at the 23rd position from the N-terminus of the α subunit" refers to an amino acid residue at the 24th from the N-terminus of the α subunit of nitrile hydratase derived from wild-type Pseudonocardia thermophila.

In an embodiment, the amino acid sequence of the mutant nitrile hydratase may be an amino acid sequence of the mutant nitrile hydratase as described in International Publication No. 2010/055666 and International Publication No. 2018/124247; or an amino acid sequence analogous to the amino acid sequence.

The method of producing the mutant nitrile hydratase of the present disclosure is not particularly limited. For example, the mutant nitrile hydratase may be produced by preparing a vector including an nucleic acid, which is represented by a nucleotide sequence encoding the amino acid sequence of the mutant nitrile hydratase; and causing the vector to generate the mutant nitrile hydratase. The details are described in the following.

### <Nucleic Acid>

The nucleic acid of the present disclosure encodes an amino acid sequence of the mutant nitrile hydratase of the present disclosure. More specifically, the nucleic acid of the present disclosure has a nucleotide sequence encoding an amino acid sequence of the mutant nitrile hydratase of the present disclosure.

Examples of the method of synthesizing a nucleotide sequence encoding an amino acid sequence of the mutant nitrile hydratase of the present disclosure include a method of introducing a mutation into a nucleotide sequence of the corresponding nitrile hydratase derived from wild-type Pseudonocardia thermophila, and a method of chemically synthesizing the total nucleotide sequence including a mutation.

Examples of the method of causing genetic mutation using a nucleotide sequence encoding nitrile hydratase derived from wild-type Pseudonocardia thermophila as a template include site directed mutagenesis (Kramer, W. and frita, H.J., Methods in Enzymology, vol. 154, p.350 (1987)); recombinant PCR (PCR Technology, Stockton Press (1989)); a method of chemically synthesizing a specific portion of a nucleic acid; a method of treating a gene with hydroxylamine; a method of exposing a strain preserving a gene to ultraviolet rays; a method of treating with a chemical such as nitrosoguanidine or nitrous acid; and a method of using a commercially available kit for introducing a mutation.

The nucleic acid of the present disclosure may be either a deoxyribonucleic acid (DNA) or a ribonucleic acid (RNA).

A gene encoding nitrile hydratase derived from wild-type Pseudonocardia thermophila is formed from a nucleotide sequence represented by SEQ ID NO: 3 and a nucleotide sequence represented by SEQ ID NO: 4.

The nucleotide sequence represented by SEQ ID NO: 3 corresponds to the amino acid sequence represented by SEQ ID NO: 1, and the nucleotide sequence represented by SEQ ID NO: 4 corresponds to the amino acid sequence represented by SEQ ID NO: 2.

The nucleic acid encoding the mutant nitrile hydratase at least has a nucleotide substitution corresponding to at least one amino acid residue substitution selected from the foregoing group (a) to (q), at a position selected from the foregoing group (a) to (q) in the nucleotide sequence represented by SEQ ID NO: 3 or SEQ ID NO: 4.

### <Vector>

The vector of the present disclosure includes the nucleic acid of the present disclosure.

Examples of the vector include those obtained by introducing the mutant nitrile hydratase of the present disclosure into a known vector. The vector of the present disclosure may be a phage vector or a plasmid vector.

### <Expression Vector>

The vector of the present disclosure is preferably an expression vector.

The expression vector is not specifically limited as long as it includes a nucleic acid represented by a nucleotide sequence encoding an amino acid sequence of the mutant nitrile hydratase of the present disclosure. According to the expression vector of the present disclosure, it is possible to produce the mutant nitrile hydratase of the present disclosure by preparing a transformant or a cell line by subjecting an arbitrary host cell to transformation, and culturing the transformant or the cell line.

As necessary, the expression vector may include a nucleotide sequence that constitutes a region other than the nucleotide sequence encoding the mutant nitrile hydratase (also simply referred to as the other region).

Examples of the other region include a control region that is required for production of the mutant nitrile hydratase and a region that is required for autonomous replicating sequence.

Examples of the control region that is required for production of the mutant nitrile hydratase include a promotor sequence (including an operator sequence that controls transcription), a ribosomal binding sequence (SD sequence), and a terminator sequence.

Examples of the promotor sequence include trp promotor of tryptophan operon and lac promoter of lactose operon derived from Escherichia coli; and PL promotor and PR promotor derived from lambda phage. It is possible to use an artificially designed or modified promotor such as tac promotor and trc promotor.

While the location of the control region in the expression vector is not particularly limited, for example, the promotor sequence and the ribosomal binding sequence are desirably located upstream of the 5' terminus of a gene encoding the mutant nitrile hydratase of the present disclosure, and the terminator sequence is desirably located downstream of the 3' terminus of a gene encoding the mutant nitrile hydratase of the present disclosure.

The respective genes of the α subunit and the β subunit of the mutant nitrile hydratase may be expressed by the control region as cistrons independent from each other, or may be expressed as polycistron by the common control region.

The expression vector may further include a nucleotide sequence that encodes a selector gene that may serve as a selected marker, from the viewpoint of facilitating the selection of the transformant.

The expression vector may express a gene that encodes a protein that engages in activation of nitrile hydratase. The protein that engages in activation of nitrile hydratase refers to a protein whose presence or non-presence of expression directly affects the chance of activation of nitrile hydratase, and representative examples thereof include a protein described in JP-A No. H11-253168 that engages in the activation of nitrile hydratase derived from Pseudonocardia thermophila (nitrile hydratase-activating protein). The sequence of the nitrile hydratase-activating protein is represented by SEQ ID NO: 36.

As for the methods for transforming the expression vector to a desired host cell, the methods for generating nitrile hydratase in the transformant, and the like, any methods and host cells that are commonly known in the field of molecular biology, biological engineering and genetic engineering may be employed, such as those described in Molecular Cloning 3rd Edition (J.Sambrook et al.; Cold Spring Harbor Laboratory Press, 2001).

### <Transformant>

The transformant of the present disclosure includes the expression vector of the present disclosure.

According to the transformant of the present disclosure, it is possible to produce the mutant nitrile hydratase by which the inhibition by an impurity with respect to the reaction to generate an amide compound using nitrile hydratase is reduced, even in the presence of the impurity.

The transformant of the present disclosure may be prepared by a known methods. For example, the transformant may be prepared by a method of constructing an expression vector including a nucleotide sequence encoding the mutant nitrile hydratase and other optional regions, and transforming the expression vector to a desired host cell. Specifically, methods that are commonly known in the field of molecular biology, biological engineering and genetic engineering may be employed, such as those described in Sambrook, J., et.al., Molecular Cloning A Laboratory Manual, 3rd Edition, Cold Spring Harbor Laboratory Press (2001).

The transformant may be prepared by incorporating a silent mutation for the purpose of enhancing the frequency of use of a codon whose frequency of use is low in the host cell, in addition to the expression vector. In that way, it may be possible to increase the amount of production of a protein derived from the mutant nitrile hydratase being incorporated in the expression vector.

The method of incorporation of a silent mutation, mutational site, type of the nucleotide for the mutation, and the like are not particularly limited as long as the method is to adjust a codon on the expression vector for a nitrile hydratase gene and a signal sequence that promotes the secretion of nitrile hydratase outside the cell, depending on the frequency of use of the codon in the host cell.

### <Production method of mutant nitrile hydratase>

The production method of a mutant nitrile hydratase of the present disclosure may be a method of producing a mutant nitrile hydratase, the method including:
growing the foregoing transformant of the present disclosure in a culture medium; and
collecting the mutant nitrile hydratase from at least one of the transformant or the culture medium.

### (Method for growing transformant)

The conditions for growing a transformant obtained by transformation with an expression vector are the same as the conditions for growing a host cell before being subjected to transformation, and may be selected from known conditions.

Examples of the host cell include procaryotic organisms (such as Escherichia coli, Bacillus subtilis and Streptomyces carbophilus), yeast and filamentous fungi.

The culture medium may be either a synthetic medium or a natural medium, as long as it includes appropriate amounts of carbon source, nitrogen source, inorganic matters and other nutrients.

The components of the culture medium may be selected from known components, for example, an appropriate combination of organic nutrient sources such as meat extract, yeast extract, malt extract, peptone, NZ amine and potato; carbon sources such as glucose, maltose, sucrose, starch and organic acids; nitrogen sources such as ammonium sulfate, urea and ammonium chloride; inorganic nutrients such as phosphoric salt, magnesium, potassium and iron; and vitamins.

In a case of growing a transformant obtained from an expression vector including a selection marker that is chemical-resistant, a culture medium containing the corresponding chemical may be used. In a case of growing a transformant obtained from an expression vector including a selection marker that is auxotrophic, a culture medium that does not contain the corresponding nutrient may be used.

The pH of the culture medium may be selected appropriately from a range of 4 to 8.

The culture method of a transformant may be an ordinary method such as shaking culture, aeration/stirred culture, continuous culture, and feeding culture.

The culture conditions may be selected appropriately depending on the type of the transformant, the culture medium or the culture method, as long as the transformant can grow and generate the mutant nitrile hydratase.

The culture temperature is preferably from 20°C and 45°C, more preferably from 24°C to 37°C, and the culture is preferably aerobically performed under the foregoing temperature.

The culture period may be selected from 1 day to 7 days so as to achieve a maximum content of a desired protein having a mutant nitrile hydratase activity.

For example, when Escherichia coli is used as the host cell, a culture medium such as LB medium or M9 medium is commonly used for the culture of the transformant. Preferably, Fe ions and Co ions are added to the culture medium as the components thereof. After inoculating, the transformant may be grown at an appropriate temperature (generally from 20°C to 50°C).

### (Collection of mutant nitrile hydratase)

The production method of a mutant nitrile hydratase preferably includes a process of collecting the mutant nitrile hydratase from at least one of the transformant or the culture medium after the culture.

The collection of the mutant nitrile hydratase after growing the transformant may be performed by a method commonly used in the art.

When the mutant nitrile hydratase is secreted outside the transformant, a crude enzyme liquid can be readily obtained by subjecting the culture product of the transformant to centrifugal separation, filtration or the like.

When the mutant nitrile hydratase is accumulated inside the transformant, a crude enzyme liquid can be obtained by collecting the transformant by centrifugal separation, suspending the collected transformant in a buffer solution, and breaking a cell wall of the transformant by a known process such as lysozyme treatment, freezing and thawing, or ultrasonic breaking.

The crude enzyme liquid including the collected mutant nitrile hydratase may be subjected to filtration with the addition of a cationic polymer and a filtration aid. The crude enzyme liquid after being subjected to filtration may be concentrated by ultrafiltration and added with an antiseptic or the like, thereby obtaining a concentrated mutant nitrile hydratase (concentrated enzyme).

When the crude enzyme liquid including the collected mutant nitrile hydratase needs to be subjected to separation and refinement, examples of the method for separation and refinement include salt precipitation using ammonium sulfate or the like, organic solvent precipitation using alcohol or the like, membrane separation such as dialysis or ultrafiltration, and known chromatographic methods such as ion exchange chromatography, reversed-phase high-pressure chromatography, affinity chromatography, and gel filtration chromatography. The methods may be combined as appropriate.

### <Production method of amide compound>

The production method of an amide compound of the present disclosure includes contacting the mutant nitrile hydratase with a nitrile compound.

The mutant nitrile hydratase of the present disclosure functions as a catalyst for a reaction to synthesize an amide compound from a nitrile compound. According to the production method of an amide compound of the present disclosure, inhibition by an impurity with respect to the reaction to generate an amide compound using nitrile hydratase is suppressed.

First, a culture fluid, a cell or a processed cell product is obtained by growing a transformant or a cell line that produces the mutant nitrile hydratase. Subsequently, the culture fluid, the cell or the processed cell product is contacted with a nitrile compound in a medium. **In** that way, the mutant nitrile hydratase comes in contact with a nitrile compound, and converts the nitrile compound into a corresponding amide compound.

**In** the present disclosure, the processed cell product refers to an extracted product or a ground product obtained from the transformant; an after-separation product such as a crude enzyme liquid obtained by separating an active fraction of nitrile hydratase from the extracted product or the ground product, or a purified product obtained by subjecting the crude enzyme liquid to purification; and an immobilized product obtained by immobilizing the extracted product, the ground product or the after-separation product by an appropriate means.

The contact is performed at a temperature at which the mutant nitrile hydratase of the present disclosure does not deactivate, preferably from 0°C to 60°C, more preferably from 15°C to 40°C.

The contact may be performed, for example, by adding a culture solution that has been used to grow the transformant or a cell line that produces the mutant nitrile hydratase of the present disclosure, directly to an aqueous solution including a nitrile compound; or by adding a bacterial body separated from the culture solution to an aqueous solution including a nitrile compound.

The pH of the aqueous solution during the reaction is preferably from 7 to 9, more preferably from 7.5 to 8.5. The nitrile compound may be present in the aqueous solution at a concentration of from 0.25% by volume to 20.0% by volume, more preferably from 2.0% by volume to 5.0% by volume, for example. The details for the culture of the transformant are the same as those mentioned in the production method of the mutant nitrile hydratase.

Specific examples of the nitrile compound are not particularly limited as long as it may be used as a substrate for the mutant nitrile hydratase of the present disclosure, and include aliphatic saturated nitriles such as acetonitrile, propionitrile, succinonitrile and adiponitrile; aliphatic unsaturated nitriles such as acrylonitrile and methacrylonitrile; aromatic nitriles such as benzonitrile and phthalodinitrile; and heterocyclic nitriles such as 3-cyanopyridine and 2-cyanopyridine.

In the method of the present disclosure, a nitrile group in a nitrile compound is converted into an amide group by way of hydration. For example, acrylonitrile can be converted into acrylamide.

Subsequently, the medium is added with activated charcoal without deactivating the mutant nitrile hydratase of the present disclosure. In that way, the amide compound is purified without terminating the reaction to synthesize an amide compound from a nitrile compound using the mutant nitrile hydratase as a catalyst.

The reaction to produce an amide compound from a nitrile compound by contacting the nitrile compound with the mutant nitrile hydratase of the present disclosure may be performed under the neutral to basic conditions, such as from pH 7 to 9, depending on the pH suitable for the nitrile hydratase. The pH may be adjusted by using a basic substance such as ammonia or a sodium hydroxide in a buffer solution, as necessary.

### Examples

The embodiments are explained in further detail by referring to the Examples. However, the present invention is in no way limited by the Examples.

In the following Examples, the "mutation site" refers to a site at which the amino acid sequence is different from an amino acid sequence of nitrile hydratase derived from wild-type Pseudonocardia thermophila, having an α subunit with an amino acid sequence represented by SEQ ID NO: 1 and a β subunit with an amino acid sequence represented by SEQ ID NO: 2.

### (Preparation of comparative nitrile hydratase derived from wild-type Pseudonocardia thermophila (C1))

In a 30 mL test tube, a LB liquid culture medium (10 mL) was prepared, and the liquid culture medium was sterilized in an autoclave at 121°C for 20 minutes. After adding ampicillin at a final concentration of 100 µg/mL, the liquid culture medium was inoculated with the cell line MT-10822, described in Patent Document 1, Example 3, by an amount of 1 platinum loop, and cultured for approximately 20 hours at 37°C, 300 rpm. Thereafter, the culture solution was placed in a centrifugal tube by an amount of 1 mL, and a bacterial body was separated by centrifugal separation (15000 rpm, 5 minutes).

Subsequently, a plasmid pPT-DB1 was prepared from the bacterial body by alkaline-SDS extraction. A transformant (C1) was obtained using the plasmid and a competent cell of Escherichia coli HB101 (Toyobo). The transformant (C1) produces nitrile hydratase (C1) as nitrile hydratase derived from wild-type Pseudonocardia thermophila.

### (Preparation of comparative mutant nitrile hydratase (C2))

A transformant that produces comparative mutant nitrile hydratase (C2) was prepared by a method described in WO 2010/055666, Comparative Example 2, using LA PCR in vitro mutagenesis Kit (Takarashuzo, hereinafter also referred to as the mutagenesis kit).

Specifically, a transformant that expresses a mutant nitrile hydratase was prepared by substituting the 92nd amino acid residue from the N-terminus in the α subunit of wild-type Pseudonocardia thermophila, from Asp to Glu. As a template, the plasmid pPT-DB1 expressing nitrile hydratase derived from wild-type Pseudonocardia thermophila was used.

In the following, a primer including a mutant sequence to be introduced, such as the sequence represented by SEQ ID NO: 5, is also referred to as a primer for mutation.

### (Preparation of mutant nitrile hydratase of the present disclosure (1))

A transformant that produces a mutant nitrile hydratase, in which the 23rd amino acid residue from the N-terminus in the α subunit represented by SEQ ID NO: 1, is substituted from Ala to Gly, was prepared using LA PCR in vitro mutagenesis Kit (Takarashuzo, hereinafter also referred to as mutagenesis kit). As a template for the PCR reaction, the plasmid pPT-DB1 expressing nitrile hydratase derived from wild-type Pseudonocardia thermophila was used.

The PCR reaction No. 1 was performed using a system (total amount: 50 µL, the composition depends on the conditions described in the mutagenesis kit) containing 50 pmol of the primer for mutation with the sequence number described in Table 5 and 50 pmol of M13 primer M4 (represented by SEQ ID NO: 33). The PCR reaction No. 1 includes 25 cycles each consisting of thermal denaturation (98°C, 15 seconds), annealing (55°C, 30 seconds) and extension (72°C, 120 seconds).

The PCR reaction No. 2 was performed using a system (total amount: 50 µL, the composition depends on the conditions described in the mutagenesis kit) including 50 pmol of MT4 primer (represented by SEQ ID NO: 34) and 50 pmol of M13 primer RV (represented by SEQ ID NO: 35) under the same conditions as the PCR reaction No. 1.

The solution after the PCR reaction No. 1 and the solution after the No. 2 (5 µL) were subjected to agarose electrophoresis (agarose concentration: 1.0 % by weight). As a result, existence of an amplified DNA product was confirmed.

After removing excessive primer and dNTP using Microcon 100 (Takarashuzo) from the solutions, TE was added to prepare TE solutions (50 µL, respectively). An annealing solution (total amount: 47.5 µL, the composition depends on the conditions described in the mutagenesis kit) containing the TE solutions (0.5 µL, respectively) was prepared and subjected to thermal denaturation (98°C, 10 seconds). Subsequently, the annealing solution was subjected to an annealing process consisting of cooling the solution to 37°C at a constant rate over 60 minutes and maintaining the solution at 37°C for 15 minutes.

After the annealing process, the solution was added with 0.5 µL of TaKaRa LA Taq, and subjected to a thermal treatment (72°C, 3 minutes) to complete the heteroduplex formation.

The resultant was added with M13 primer M4 (50 pmol) and M13 primer RV (50 pmol), and the total amount was adjusted to 50 µL. Thereafter, the solution was subjected to the PCR reaction No. 3 including 25 cycles each consisting of thermal denaturation (98°C, 15 seconds), annealing (55°C, 30 seconds) and extension (72°C, 120 seconds).

The solution after the PCR reaction No. 3 (5 µL) was subjected to agarose electrophoresis (Type VII low-melting agarose, Sigma-Aldrich, agarose concentration: 0.8 % by weight). As a result, existence of an amplified DNA product, with a size of approximately 2 kb, was confirmed.

Subsequently, a portion corresponding to the DNA fragment of approximately 2 kb was cut out from the agarose gel, and the portion (approximately 0.1 g) was pulverized and suspended in a TE solution (1 mL). The TE solution was kept warm (55°C, 1 hour) to allow the agarose to completely melt.

The TE solution after melting the agarose was subjected to phenol/chloroform extraction and ethanol precipitation for purification of the DNA fragment, and finally dissolved in 10 µL of TE.

The purified DNA fragment of approximately 2 kb was cleaved with restriction enzymes EcoRI and Hind III. The solution after the treatment with restriction enzymes was subjected to phenol/chloroform extraction and ethanol precipitation for purification of the DNA fragment, and finally dissolved in 10 µL of TE.

In a similar manner, the nitrile hydratase-expressing plasmid pPT-DB1 was cleaved with restriction enzymes EcoRI and Hind III, subjected to agarose gel electrophoresis (Type VII low-melting agarose, Sigma-Aldrich, agarose concentration: 0.7 % by weight), and a portion corresponding to the DNA fragment in a size of approximately 2.7 kb was cut out from the agarose gel. The portion of the agarose gel (approximately 0.1 g) was pulverized and suspended in a TE solution (10 µL). The TE solution was kept warm (55 °C, 1 hour) to allow the agarose to completely melt.

The TE solution after melding the agarose was subjected to phenol/chloroform extraction and ethanol precipitation for purification of the DNA fragment, and finally dissolved in 10 µL of TE.

The DNA fragment of approximately 2 kb and the DNA fragment of approximately 2.7 kb obtained by the forgoing process were ligated using a DNA ligation kit (Takarashuzo). Thereafter, the transformant (1) was obtained by transformation using a competent cell of Escherichia coli HB101 (Toyobo).

Further, a plasmid was prepared from the bacterial body by alkaline-SDS extraction, and the nucleotide sequence was determined at a portion corresponding to a gene of nitrile hydratase with a DNA sequencer. It was confirmed that the plasmid pPT-DB1 had the 23rd amino acid residue from the N-terminus in the α subunit being substituted from Ala to Gly. The transformant (1) produces a mutant nitrile hydratase (1) having the mutation as described in Table 5.

### (Preparation of mutant nitrile hydratases (2) to (17) of the present disclosure)

Transformants (2) to (17), expressing a mutant nitrile hydratase having an amino acid residue substitution as described in Table 5, were prepared in the same manner as the preparation of the transformant (1) using the primers of SEQ ID NO: 6 to SEQ ID NO: 21 instead of the primer of SEQ ID NO: 5.

The transformants (2) to (17) produce the nitrile hydratases (2) to (17) having the mutations as described in Table 5.

### (Preparation of mutant nitrile hydratase (18) to (28) of the present disclosure)

Transformant (18), expressing a mutant nitrile hydratase having an amino acid residue substitution as described in Table 6, was prepared by obtaining a plasmid in the same manner as the preparation of the transformant (1). Specifically, the transformant (18) was prepared using a plasmid obtained from the transformant (9) instead of pPT-DB1 as a template, and using the primer for mutation of SEQ ID NO: 18. The transformant (18) produces the nitrile hydratase (18) having the mutation as described in Table 6.

In a similar manner, the nitrile hydratase (19) was prepared using a plasmid obtained from the transformant (9) and the primer for mutation of SEQ ID NO: 15;
the nitrile hydratase (20) was prepared using a plasmid obtained from the transformant (13) and the primer for mutation of SEQ ID NO: 11;
the nitrile hydratase (21) was prepared using a plasmid obtained from the transformant (10) and the primer for mutation of SEQ ID NO: 18;
the nitrile hydratase (22) was prepared using a plasmid obtained from the transformant (9) and the primer for mutation of SEQ ID NO: 11;
the nitrile hydratase (23) was prepared using a plasmid obtained from the transformant (16) and the primer for mutation of SEQ ID NO: 21;
the nitrile hydratase (24) was prepared using a plasmid obtained from the transformant (1) and the primer for mutation of SEQ ID NO: 7;
the nitrile hydratase (25) was prepared using a plasmid obtained from the transformant (6) and the primer for mutation of SEQ ID NO: 19;
the nitrile hydratase (26) was prepared using a plasmid obtained from the transformant (17) and the primer for mutation of SEQ ID NO: 22;
the nitrile hydratase (27) was prepared using a plasmid obtained from the transformant (5) and the primer for mutation of SEQ ID NO: 23; and
the nitrile hydratase (28) was prepared using a plasmid obtained from the transformant (7) and the primer for mutation of SEQ ID NO: 24.

### (Preparation of mutant nitrile hydratases (29) to (37) of the present disclosure)

Transformant (29), expressing a mutant nitrile hydratase having an amino acid residue substitution as described in Table 7, was prepared in the same manner as the preparation of the transformant (1). Specifically, the transformant (29) was prepared using a plasmid obtained from the transformant (18) instead of pPT-DB1 as a template, and using the primer for mutation of SEQ ID NO: 11. The transformant (29) produces the nitrile hydratase (29) having the mutation as described in Table 7.

In a similar manner, the nitrile hydratase (30) was prepared using a plasmid obtained from the transformant (21) and the primer for mutation of SEQ ID NO: 13;
the nitrile hydratase (31) was prepared using a plasmid obtained from the transformant (20) and the primer for mutation of SEQ ID NO: 6;
the nitrile hydratase (32) was prepared using a plasmid obtained from the transformant (20) and the primer for mutation of SEQ ID NO: 16;
the nitrile hydratase (33) was prepared using a plasmid obtained from the transformant (20) and the primer for mutation of SEQ ID NO: 18;
the nitrile hydratase (34) was prepared using a plasmid obtained from the transformant (9), a plasmid obtained from a transformant that is obtained from the primer for mutation of SEQ ID NO: 16, and the primer for mutation of SEQ ID NO: 17,
the nitrile hydratase (35) was prepared using a plasmid obtained from the transformant (23) and the primer for mutation of SEQ ID NO: 11;
the nitrile hydratase (36) was prepared using a plasmid obtained from the transformant (15), a plasmid obtained from a transformant that is obtained from the primer for mutation of SEQ ID NO: 25, and the primer for mutation of SEQ ID NO: 26; and
the nitrile hydratase (37) was prepared using a plasmid obtained from the transformant (8), a plasmid obtained from a transformant that is obtained from the primer for mutation of SEQ ID NO: 27, and the primer for mutation of SEQ ID NO: 28.

### (Preparation of mutant nitrile hydratases (38) to (46) of the present disclosure)

Transformant (38), expressing a mutant nitrile hydratase having an amino acid residue substitution as described in Table 8, was prepared in the same manner as the preparation of the transformant (1). Specifically, the transformant (38) was prepared using a plasmid obtained from the transformant (30) instead of pPT-DB1 as a template, and using the primer for mutation of SEQ ID NO: 11. The transformant (38) produces the nitrile hydratase (38) having the mutation as described in Table 8.

In a similar manner, the nitrile hydratase (39) was prepared using a plasmid obtained from the transformant (29) and the primer for mutation of SEQ ID NO: 17;
the nitrile hydratase (40) was prepared using a plasmid obtained from the transformant (34) and the primer for mutation of SEQ ID NO: 11;
the nitrile hydratase (41) was prepared using a plasmid obtained from the transformant (35) and the primer for mutation of SEQ ID NO: 19;
the nitrile hydratase (42) was prepared using a plasmid obtained from the transformant (8), a plasmid obtained from a transformant that is obtained from the primer for mutation of SEQ ID NO: 22, a plasmid obtained from a transformant that is obtained from the primer for mutation of SEQ ID NO: 27, and the primer for mutation of SEQ ID NO: 32;
the nitrile hydratase (43) was prepared using a plasmid obtained from the transformant (40) and the primer for mutation of SEQ ID NO: 18;
the nitrile hydratase (44) was prepared using a plasmid obtained from the transformant (40) and the primer for mutation of SEQ ID NO: 29;
the nitrile hydratase (45) was prepared using a plasmid obtained from the transformant (35), a plasmid obtained from a transformant that is obtained from the primer for mutation of SEQ ID NO: 27, and the primer for mutation of SEQ ID NO: 28; and
the nitrile hydratase (46) was prepared using a plasmid obtained from the transformant (1), a plasmid obtained from a transformant that is obtained from the primer for mutation of SEQ ID NO: 11, a plasmid obtained from a transformant that is obtained from the primer for mutation of SEQ ID NO: 29, a plasmid obtained from a transformant that is obtained from the primer for mutation of SEQ ID NO: 30, and the primer for mutation of SEQ ID NO: 31.

In Table 5 to Table 10, the column "template" indicates the number of the tansformant from which the plasmid is obtained; the column "primer for mutation" indicates the sequence number of the primer used as the primer for mutation; the column "amino acid residue before mutation" indicates the amino acid residue before the mutation at a mutation site indicated in the column "mutation site", i.e., the amino acid residue of nitrile hydratase derived from wild-type Pseudonocardia thermophila (C1); and the column "amino acid residue after mutation" indicates the amino acid residue after the mutation at a mutation site indicated in the column "mutation site".

### [Examples 1-1 to 1-17, 2-1 to 2-11, 3-1 to 3-9, 4-1 to 4-9, B2-B7, C1 to C5, Comparative Examples 1, 2-1, 2-2, 3 to 4, and Reference Examples 1 to 3]

### <Evaluation on suppression of inhibition of reaction by impurity>

The reaction to generate an amide compound in the presence of an impurity was caused using the transformant that produces nitrile hydratase of respective numbers.

In a test tube, a LB liquid culture medium (5 mL) containing ion (II) sulfate heptahydrate (40 µm/mL) and cobalt (II) chloride dihydrate (10 µm/mL) was prepared, and the liquid culture medium was sterilized in an autoclave at 121°C for 20 minutes. After adding ampicillin at a final concentration of 100 µg/mL, the liquid culture medium was inoculated with the transformant by an amount of 1 platinum loop, and was cultured for approximately 20 hours at 37°C, 200 rpm. Thereafter, the culture medium was placed in a centrifugal tube by an amount of 1000 µL, and a bacterial body was separated by centrifugal separation. The bacterial body was suspended in 780 µL of 50 mM tris-HCl aqueous solution (pH 8.0) containing acrolein by an amount of 1000 ppm by mass. The suspension was added with 20 µL of acrylonitrile, and caused to react for 60 minutes while moderately stirring at a temperature indicated in Table 5 to Table 10.

After the reaction, the amount of an amide compound (acrylamide) included in the reaction solution was quantified by HPLC under the following conditions.

In order to determine the relative intensity of enzymatic activity of the mutant nitrile hydratase to produce an amide compound from a nitrile compound, the "amount of amide compound obtained with nitrile hydratase of respective numbers in the presence of impurity (X)" was contrasted with the "amount of amide compound obtained in Comparative Example 1 with nitrile hydratase (C1) derived from wild-type Pseudonocardia thermophila in the presence of impurity (Y)", thereby determining the suppression degree of inhibition of reaction by impurity (X/Y). For example, the suppression degree of inhibition of reaction by impurity in Comparative Example 1 is 1.000, since the same results are contrasted with each other.

In Reference Example 1, the "amount of amide compound obtained with nitrile hydratase (1) in the absence of impurity" was contrasted with the "amount of amide compound obtained in Example 1-1 with nitrile hydratase (1) in the presence of impurity".

In Reference Example 2, the "amount of amide compound obtained with nitrile hydratase (C1) in the absence of impurity" was contrasted with the "amount of amide compound obtained in Comparative Example 1 with nitrile hydratase (C1) in the presence of impurity".

In Reference Example 3, the "amount of amide compound obtained with nitrile hydratase (C2) in the absence of impurity" was contrasted with the "amount of amide compound obtained in Comparative Example 2-1 with nitrile hydratase (C2) in the presence of impurity".

The results are shown in the Tables. The reaction and analysis were conducted at least three times for each number of nitrile hydratase, in order to correct the dispersion in data that may be caused by procedures such as dispensing.

### <Analysis conditions>

Analytical instrument: HPLC, JASCO Corporation
Column: YMC Pack ODS-A (150 × 6.00mm)
Analytical temperature: 40°C
Mobile phase: 3% acetonitrile, 10 mM phosphoric acid

**Table 5**

| | Nitrile hydratase | | | | | Reaction Temperatu re [°C] | Acrolein concentrati on [ppm] | Suppressio n degree of inhibition of reaction by impurity |
|---|---|---|---|---|---|---|---|---|
| | Nitrile hydratase No. | Primer for mutation | Mutation site | Amino acid residue before mutation | Amino acid residue after mutation | | | |
| Ref. Example 1 | 1 | SEQ ID No: 5 | α23 | Ala | Gly | 20 | 0 | 2.013 |
| Ref. Example 2 | C1 | - | - | - | - | 20 | 0 | 1.994 |
| Ref. Example 3 | C2 | - | α92 | Asp | Glu | 20 | 0 | 2.263 |
| Comp. Example 1 | C1 | - | - | - | - | 20 | 1000 | 1.000 |
| Comp. Example 2-1 | C2 | - | α92 | Asp | Glu | 20 | 1000 | 0.960 |
| Example 1-1 | 1 | SEQ ID No: 5 | α23 | Ala | Gly | 20 | 1000 | 1.125 |
| Example 1-2 | 2 | SEQ ID No: 6 | α105 | Val | Met | 20 | 1000 | 1.117 |
| Example 1-3 | 3 | SEQ ID No: 7 | α142 | Leu | Cys | 20 | 1000 | 1.117 |
| Example 1-4 | 4 | SEQ ID No: 8 | α145 | Glu | Asp | 20 | 1000 | 1.112 |
| Example 1-5 | 5 | SEQ ID No: 9 | α152 | Pro | Gly | 20 | 1000 | 1.166 |
| Example 1-6 | 6 | SEQ ID No: 10 | α153 | Pro | Arg | 20 | 1000 | 1.139 |
| Example 1-7 | 7 | SEQ ID No: 11 | α180 | Gly | Val | 20 | 1000 | 1.188 |
| Example 1-8 | 8 | SEQ ID No: 12 | β89 | Glu | Leu | 20 | 1000 | 1.148 |
| Example 1-9 | 9 | SEQ ID No: 13 | β122 | Ala | Ile | 20 | 1000 | 1.185 |
| Example 1-10 | 10 | SEQ ID No: 14 | β149 | Thr | Gln | 20 | 1000 | 1.185 |
| Example 1-11 | 11 | SEQ ID No: 15 | β153 | Lys | Arg | 20 | 1000 | 1.174 |
| Example 1-12 | 12 | SEQ ID No: 16 | β189 | Cys | Ser | 20 | 1000 | 1.141 |
| Example 1-13 | 13 | SEQ ID No: 17 | β207 | Glu | Gly | 20 | 1000 | 1.117 |
| Example 1-14 | 14 | SEQ ID No: 18 | β232 | Ala | Trp | 20 | 1000 | 1.170 |
| Example 1-15 | 15 | SEQ ID No: 19 | β233 | Ala | Glu | 20 | 1000 | 1.144 |
| Example 1-16 | 16 | SEQ ID No: 20 | α188 | Thr | Lys | 20 | 1000 | 1.117 |
| Example 1-17 | 17 | SEQ ID No: 21 | β50 | Glu | Gly | 20 | 1000 | 1.106 |

**Table 6**

| | Nitrile hydratase | | | | | | Reaction Temperat ure [°C] | Acrolein concentra tion [ppm] | Suppression degree of inhibition of reaction by impurity |
|---|---|---|---|---|---|---|---|---|---|
| | Nitrile hydratase No. | Template | Primer for mutation | Mutation site | Amino acid residue before mutation | Amino acid residue after mutation | | | |
| Example 2-1 | 18 | 9 | 18 | β122 | Ala | Ile | 20 | 1000 | 1.269 |
| | | | | β232 | Ala | Trp | | | |
| Example 2-2 | 19 | 9 | 15 | β122 | Ala | Ile | 20 | 1000 | 1.251 |
| | | | | β153 | Lys | Arg | | | |
| Example 2-3 | 20 | 13 | 11 | β207 | Glu | Pro | 20 | 1000 | 1.255 |
| | | | | α180 | Gly | Val | | | |
| Example 2-4 | 21 | 10 | 18 | β149 | Thr | Gln | 20 | 1000 | 1.216 |
| | | | | β232 | Ala | Trp | | | |
| Example 2-5 | 22 | 9 | 11 | α180 | Gly | Val | 20 | 1000 | 1.284 |
| | | | | β122 | Ala | Ile | | | |
| Example 2-6 | 23 | 16 | 21 | α188 | Thr | Lys | 20 | 1000 | 1.212 |
| | | | | β50 | Glu | Gly | | | |
| Example 2-7 | 24 | 1 | 7 | α23 | Ala | Gly | 20 | 1000 | 1.268 |
| | | | | α142 | Leu | Cys | | | |
| Example 2-8 | 25 | 6 | 19 | β153 | Lys | Val | 20 | 1000 | 1.297 |
| | | | | β233 | Ala | Glu | | | |
| Example 2-9 | 26 | 17 | 22 | β50 | Glu | Gly | 20 | 1000 | 1.212 |
| | | | | β76 | Thr | Cys | | | |
| Example 2-10 | 27 | 5 | 23 | α152 | Pro | Gly | 20 | 1000 | 1.282 |
| | | | | α141 | Gln | Arg | | | |
| Example 2-11 | 28 | 7 | 24 | α180 | Gly | Val | 20 | 1000 | 1.278 |
| | | | | β50 | Glu | Gly | | | |

**Table 7**

| | Nitrile hydratase | | | | | | Reaction Temperatu re [°C] | Acrolein concentrati on [ppm] | Suppressio n degree of inhibition of reaction by impurity |
|---|---|---|---|---|---|---|---|---|---|
| | Nitrile hydratase No. | Template | Primer for mutation | Mutation site | Amino acid residue before mutation | Amino acid residue after mutation | | | |
| Example 3-1 | 29 | 18 | 11 | β122 | Ala | Leu | 20 | 1000 | 1.223 |
| | | | | β232 | Ala | Pro | | | |
| | | | | α180 | Gly | Val | | | |
| Example 3-2 | 30 | 21 | 13 | β122 | Ala | Ile | 20 | 1000 | 1.227 |
| | | | | β149 | Thr | Gln | | | |
| | | | | β232 | Ala | Trp | | | |
| Example 3-3 | 31 | 20 | 6 | β207 | Glu | Gly | 20 | 1000 | 1.255 |
| | | | | α105 | Val | Met | | | |
| | | | | α180 | Gly | Val | | | |
| Example 3-4 | 32 | 20 | 16 | β207 | Glu | Gly | 20 | 1000 | 1.264 |
| | | | | β189 | Cys | Ser | | | |
| | | | | α180 | Gly | Val | | | |
| Example 3-5 | 33 | 20 | 18 | β207 | Glu | Gly | 20 | 1000 | 1.260 |
| | | | | β232 | Ala | Gly | | | |
| | | | | α180 | Gly | Val | | | |
| Example 3-6 | 34 | 9 | 16,17 | β122 | Ala | Ile | 20 | 1000 | 1.290 |
| | | | | β189 | Cys | Ser | | | |
| | | | | β207 | Glu | Gly | | | |
| Example 3-7 | 35 | 23 | 11 | α188 | Thr | Lys | 20 | 1000 | 1.205 |
| | | | | β50 | Glu | Gly | | | |
| | | | | α180 | Gly | Val | | | |
| Example 3-8 | 36 | 15 | 25,26 | β233 | Ala | Glu | 20 | 1000 | 1.279 |
| | | | | α105 | Val | Met | | | |
| | | | | α185 | Glu | Ala | | | |
| Example 3-9 | 37 | 8 | 27,28 | β89 | Glu | Leu | 20 | 1000 | 1.235 |
| | | | | β146 | Arg | Gln | | | |
| | | | | β160 | Arg | Gln | | | |

**Table 8**

| | Nitrile hydratase | | | | | | Reaction Temperat ure [°C] | Acrolein concentra tion [ppm] | Suppression degree of inhibition of reaction by impurity |
|---|---|---|---|---|---|---|---|---|---|
| | Nitrile hydratase No. | Template | Primer for mutation | Mutation site | Amino acid residue before mutation | Amino acid residue after mutation | | | |
| Example 4-1 | 38 | 30 | 11 | β122 | Ala | Leu | 20 | 1000 | 1.281 |
| | | | | β149 | Thr | Gln | | | |
| | | | | β232 | Ala | Cys | | | |
| | | | | α180 | Gly | Val | | | |
| Example 4-2 | 39 | 29 | 17 | β122 | Ala | Ile | 20 | 1000 | 1.300 |
| | | | | β207 | Glu | Gly | | | |
| | | | | β232 | Ala | Trp | | | |
| | | | | α180 | Gly | Val | | | |
| Example 4-3 | 40 | 34 | 11 | β122 | Ala | Ile | 20 | 1000 | 1.290 |
| | | | | β189 | Cys | Ser | | | |
| | | | | β207 | Glu | Gly | | | |
| | | | | α180 | Gly | Val | | | |
| Example 4-4 | 41 | 35 | 19 | α188 | Thr | Lys | 20 | 1000 | 1.266 |
| | | | | β50 | Glu | Gly | | | |
| | | | | α180 | Gly | Val | | | |
| | | | | β233 | Ala | Glu | | | |
| Example 4-5 | 42 | 8 | 22,27, 32 | β89 | Glu | Leu | 20 | 1000 | 1.268 |
| | | | | β76 | Thr | Cys | | | |
| | | | | β88 | Leu | Arg | | | |
| | | | | β146 | Arg | Gln | | | |
| Example 4-6 | 43 | 40 | 18 | β122 | Ala | Ile | 20 | 1000 | 1.252 |
| | | | | β189 | Cys | Ser | | | |
| | | | | β207 | Glu | Gly | | | |
| | | | | β232 | Ala | Trp | | | |
| | | | | α180 | Gly | Val | | | |
| Example 4-7 | 44 | 40 | 29 | β122 | Ala | Ile | 20 | 1000 | 1.245 |
| | | | | β176 | Tyr | Cys | | | |
| | | | | β189 | Cys | Ser | | | |
| | | | | β207 | Glu | Gly | | | |
| | | | | α180 | Gly | Val | | | |
| Example 4-8 | 45 | 35 | 27,28 | α188 | Thr | Lys | 20 | 1000 | 1.272 |
| | | | | β50 | Glu | Gly | | | |
| | | | | α180 | Gly | Val | | | |
| | | | | β146 | Arg | Gln | | | |
| | | | | β160 | Arg | Gln | | | |
| Example 4-9 | 46 | 1 | 11,29, 30,31 | α23 | Ala | Gly | 20 | 1000 | 1.237 |
| | | | | α180 | Gly | Val | | | |
| | | | | β83 | Thr | His | | | |
| | | | | β108 | Glu | Arg | | | |
| | | | | β176 | Tyr | Cys | | | |

**Table 9**

| | Nitrile hydratase | | | | Reaction Temperat ure [°C] | Acrolein concentrat ion [ppm] | Suppression degree of inhibition of reaction by impurity |
|---|---|---|---|---|---|---|---|
| | Nitrile hydratase No. | Mutation site | Amino acid residue before mutation | Amino acid residue after mutation | | | |
| Example 1-1 | 1 | α23 | Ala | Gly | 20 | 1000 | 1.125 |
| Example B2 | 9 | β122 | Ala | Ile | 20 | 7.0 | 1.142 |
| Example B3 | 21 | β149 | Thr | Gln | 20 | 4.0 | 1.208 |
| | | β232 | Ala | Trp | | | |
| Example B4 | 35 | α188 | Thr | Lys | 20 | 2.2 | 1.213 |
| | | β50 | Glu | Gly | | | |
| | | α180 | Gly | Val | | | |
| Example B5 | 33 | β207 | Glu | Gly | 20 | 1.4 | 1.237 |
| | | β232 | Ala | Gly | | | |
| | | α180 | Gly | Val | | | |
| Example B6 | 36 | β233 | Ala | Glu | 20 | 1.2 | 1.245 |
| | | α105 | Val | Met | | | |
| | | α185 | Glu | Ala | | | |
| Example B7 | 38 | β122 | Ala | Leu | 20 | 1.3 | 1.240 |
| | | β149 | Thr | Gln | | | |
| | | β232 | Ala | Cys | | | |
| | | α180 | Gly | Val | | | |

**Table 10**

| | Nitrile hydratase | | | | Reaction Temperatu re [°C] | Acrolein concentrati on [ppm] | Suppressio n degree of inhibition of reaction by impurity |
|---|---|---|---|---|---|---|---|
| | Nitrile hydratase No. | Mutation site | Amino acid residue before mutation | Amino acid residue after mutation | | | |
| Comp. Example 1 | C1 | - | - | - | 20 | 1000 | 1.000 |
| Comp. Example 2-2 | C2 | α92 | Asp | Glu | 20 | 1000 | 0.947 |
| Comp. Example 3 | C1 | - | - | - | 30 | 1000 | 0.783 |
| Comp. Example 4 | C2 | α92 | Asp | Glu | 30 | 1000 | 0.752 |
| Example C1 | 9 | β122 | Ala | Ile | 30 | 1000 | 1.120 |
| Example C2 | 22 | α180 | Gly | Val | 30 | 1000 | 1.210 |
| | | β122 | Ala | Ile | | | |
| Example C3 | 35 | α188 | Thr | Lys | 30 | 1000 | 1.230 |
| | | β50 | Glu | Gly | | | |
| | | α180 | Gly | Val | | | |
| Example C4 | 34 | β122 | Ala | Ile | 30 | 1000 | 1.320 |
| | | β189 | Cys | Ser | | | |
| | | β207 | Glu | Gly | | | |
| Example C5 | 40 | β122 | Ala | Ile | 30 | 1000 | 1.450 |
| | | β189 | Cys | Ser | | | |
| | | β207 | Glu | Gly | | | |
| | | α180 | Gly | Val | | | |

In Table 5, as shown in the comparison between Comparative Example 1, in which the reaction was caused in the presence of the impurity, and Reference Example 2, in which the reaction was caused in the absence of the impurity, the enzymatic activity of wild-type nitrile hydratase (C1) is lower in the case with the impurity than in the case without the impurity, indicating that the reaction is inhibited by the impurity.

Further, as shown in the comparison between Comparative Example 2-1, in which the reaction was caused in the presence of the impurity, and Reference Example 3, in which the reaction was caused in the absence of the impurity, the enzymatic activity of mutant nitrile hydratase for comparison (C2) is lower in the case with the impurity than in the case without the impurity, indicating that the reaction is inhibited by the impurity.

Contrary to the forgoing results of wild-type nitrile hydratase (C1) and mutant nitrile hydratase for comparison (C2), the mutant nitrile hydratases of the present disclosure exhibit a small difference in enzymatic activity between the case in which reaction was caused with the impurity and the case in which reaction was caused without the impurity.

More specifically, the difference between the "suppression degree of inhibition of reaction by impurity" in Example 1-1 and the "suppression degree of inhibition of reaction by impurity" in Reference Example 1 (2.013 - 1.125 = 0.888) is smaller than the difference between the " suppression degree of inhibition of reaction by impurity" in Comparative Example 1 and the " suppression degree of inhibition of reaction by impurity" in Reference Example 2 (1.994 - 1.000 = 0.994).

Further, the difference between the "suppression degree of inhibition of reaction by impurity" in Example 1-1 and the "suppression degree of inhibition of reaction by impurity" in Reference Example 1 (2.013 - 1.125 = 0.888) is smaller than the difference between the "suppression degree of inhibition of reaction by impurity" in Comparative Example 2-1 and the "suppression degree of inhibition of reaction by impurity" in Reference Example 3 (2.263 - 0.960 = 1.303).

Accordingly, the inhibition by the impurity with respect to a reaction to generate an amide compound using nitrile hydratase is reduced by the mutant nitrile hydratase of the present disclosure, even in the presence of the impurity.

As shown in Tables 5 to 10, the mutant nitrile hydratases of the Examples, with amino acid residue substitutions of the group consisting of (a) to (q) performed on nitrile hydratase derived from wild-type Pseudonocardia thermophila, are more likely to reduce the inhibition by the impurity with respect to a reaction to generate an amide compound using nitrile hydratase, even in the presence of the impurity, as compared with the nitrile hydratase derived from wild-type Pseudonocardia thermophila of Comparative Example 1 or the mutant nitrile hydratase of Comparative Example 2-1.

As shown in Table 9, the mutant nitrile hydratases of the Examples, with amino acid residue substitutions of the group consisting of (a) to (q) performed on nitrile hydratase derived from wild-type Pseudonocardia thermophila, reduce the inhibition by the impurity with respect to a reaction to generate an amide compound using nitrile hydratase, even in the presence of the impurity at a concentration of less than 1000 ppm (for example, less than 10 ppm).

As shown in Table 10, the mutant nitrile hydratases of the Examples, with amino acid residue substitutions of (i) and the group consisting of (g), (l) and (m) performed on nitrile hydratase derived from wild-type Pseudonocardia thermophila, produce an amide compound in an efficient manner without lowering the enzymatic activity, even under higher temperature (30°C) than room temperature (20°C). Accordingly, the mutant nitrile hydratases of the Examples, with amino acid residue substitutions of (i) and the group consisting of (g), (l) and (m) performed on nitrile hydratase derived from wild-type Pseudonocardia thermophila, exhibit excellent heat resistance.

The disclosure of Japanese Patent Application No. 2022-106069 is incorporated herein by reference in its entirety. All publications, patent applications, and technical standards mentioned in the present specification are incorporated herein by reference to the same extent as if each individual publication, patent application, or technical standard was specifically and individually indicated to be incorporated by reference.

## Claims

1. A mutant nitrile hydratase, being a nitrile hydratase having an α subunit with a sequence identity of 90% or more with respect to an amino acid sequence represented by SEQ ID NO: 1 and a β subunit with a sequence identity of 90% or more with respect to an amino acid sequence represented by SEQ ID NO: 2, the nitrile hydratase comprising at least one substitution of an amino acid residue selected from the group consisting of the following (a) to (q):
(a) a substitution to Gly from Ala as an amino acid residue corresponding to a 23rd position from an N-terminus of SEQ ID NO: 1 in the α subunit;
(b) a substitution to Met from Val as an amino acid residue corresponding to a 105th position from the N-terminus of SEQ ID NO: 1 in the α subunit;
(c) a substitution to Cys from Leu as an amino acid residue corresponding to a 142th position from the N-terminus of SEQ ID NO: 1 in the α subunit;
(d) a substitution to Asp from Glu as an amino acid residue corresponding to a 145th position from the N-terminus of SEQ ID NO: 1 in the α subunit;
(e) a substitution to Gly from Pro as an amino acid residue at a 152nd position from the N-terminus of SEQ ID NO: 1 in the α subunit;
(f) a substitution to Arg or Val from Pro as an amino acid residue corresponding to a 153rd position from the N-terminus of SEQ ID NO: 1 in the α subunit;
(g) a substitution to Val from Gly as an amino acid residue corresponding to a 180th position from the N-terminus of SEQ ID NO: 1 in the α subunit;
(h) a substitution to Leu from Glu as an amino acid residue corresponding to a 89th position from an N-terminus of SEQ ID NO: 2 in the β subunit;
(i) a substitution to Ile, Leu or Thr from Ala as an amino acid residue corresponding to a 122nd position from the N-terminus of SEQ ID NO: 2 in the β subunit;
(j) a substitution to Gln from Thr as an amino acid residue corresponding to a 149th position from the N-terminus of SEQ ID NO: 2 in the β subunit;
(k) a substitution to Val, Arg or Pro from Lys as an amino acid residue corresponding to a 153rd position from the N-terminus of SEQ ID NO: 2 in the β subunit;
(l) a substitution to Ser from Cys as an amino acid residue corresponding to a 189th position from the N-terminus of SEQ ID NO: 2 in the β subunit;
(m) a substitution to Pro, Ser or Gly from Glu as an amino acid residue corresponding to a 207th position from the N-terminus of SEQ ID NO: 2 in the β subunit;
(n) a substitution to Trp, Ser, Pro, Phe, Ile, Arg, Cys or Gly from Ala as an amino acid residue corresponding to a 232nd position from the N-terminus of SEQ ID NO: 2 in the β subunit;
(o) a substitution to Glu or Gln from Ala as an amino acid residue corresponding to a 233rd position from the N-terminus of SEQ ID NO: 2 in the β subunit;
(p) a substitution to Lys from Thr as an amino acid residue corresponding to a 188th position from the N-terminus of SEQ ID NO: 1 in the α subunit; and
(q) a substitution to Gly from Glu as an amino acid residue corresponding to a 50th position from the N-terminus of SEQ ID NO: 2 in the β subunit.

2. The mutant nitrile hydratase according to claim 1, comprising at least two substitutions of an amino acid residue selected from the group consisting of (a) to (q).

3. The mutant nitrile hydratase according to claim 1 or claim 2, comprising at least one substitution of an amino acid residue selected from the group consisting of (i), (g), (j) and (n).

4. The mutant nitrile hydratase according to claim 1 or claim 2, comprising at least one substitution of an amino acid residue selected from the group consisting of (i), (g), (l) and (m).

5. The mutant nitrile hydratase according to claim 1 or claim 2, comprising at least one substitution of an amino acid residue selected from the group consisting of (g), (p) and (q).

6. The mutant nitrile hydratase according to claim 1 or claim 2, wherein the amino acid sequence of the α subunit further comprises at least one substitution of an amino acid residue selected from the group consisting of the following (A1) to (A6):
(A1) a substitution to Val from Arg as an amino acid residue corresponding to a 20th position from the N-terminus of SEQ ID NO: 1 in the α subunit;
(A2) a substitution to Arg from Gln as an amino acid residue corresponding to a 141st position from the N-terminus of SEQ ID NO: 1 in the α subunit;
(A3) a substitution to Ala from Glu as an amino acid residue corresponding to a 185th position from the N-terminus of SEQ ID NO: 1 in the α subunit;
(A4) a substitution to Arg from Ala as an amino acid residue corresponding to a 187th position from the N-terminus of SEQ ID NO: 1 in the α subunit;
(A5) a substitution to Ala from Pro as an amino acid residue corresponding to a 200th position from the N-terminus of SEQ ID NO: 1 in the α subunit; and
(A6) a substitution to Leu from Val as an amino acid residue corresponding to a 204th position from the N-terminus of SEQ ID NO: 1 in the α subunit.

7. The mutant nitrile hydratase according to claim 1 or claim 2, wherein the amino acid sequence of the β subunit further comprises at least one substitution of an amino acid residue selected from the group consisting of the following (B1) to (B9):
(B1) a substitution to Ile from Thr as an amino acid residue corresponding to a 40th position from the N-terminus of SEQ ID NO: 2 in the β subunit;
(B2) a substitution to Pro from Arg as an amino acid residue corresponding to a 42nd position from the N-terminus of SEQ ID NO: 2 in the β subunit;
(B3) a substitution to Cys from Thr as an amino acid residue corresponding to a 76th position from the N-terminus of SEQ ID NO: 2 in the β subunit;
(B4) a substitution to His from Thr as an amino acid residue corresponding to a 83rd position from the N-terminus of SEQ ID NO: 2 in the β subunit;
(B5) a substitution to Arg, Pro or Ser from Leu as an amino acid residue corresponding to a 88th position from the N-terminus of SEQ ID NO: 2 in the β subunit;
(B6) a substitution to Gln or Thr from Arg as an amino acid residue corresponding to a 146th position from the N-terminus of SEQ ID NO: 2 in the β subunit;
(B7) a substitution to Gln or Ile from Arg as an amino acid residue corresponding to a 160th position from the N-terminus of SEQ ID NO: 2 in the β subunit;
(B8) a substitution Arg from Glu as an amino acid residue corresponding to a 108th position from the N-terminus of SEQ ID NO: 2 in the β subunit; and
(B9) a substitution to Cys from Tyr as an amino acid residue corresponding to a 176th position from the N-terminus of SEQ ID NO: 2 in the β subunit.

8. A nucleic acid, encoding the mutant nitrile hydratase according to claim 1 or claim 2.

9. A vector, comprising the nucleic acid according to claim 8.

10. The vector according to claim 9, being an expression vector.

11. A transformant, comprising the expression vector according to claim 10.

12. A method of producing a mutant nitrile hydratase, the method comprising:
growing the transformant according to claim 11 in a culture medium; and
collecting the mutant nitrile hydratase according to claim 1 or claim 2 from at least one of the transformant or the culture medium.

13. A mutant nitrile hydratase obtained by the production method according to claim 12.

14. A method of producing an amide compound, the method comprising contacting the mutant nitrile hydratase according to claim 1 or claim 2 with a nitrile compound.
